# EUROPÄISCHE PATENTSCHRIFT

(11) **EP 3 092 976 B1**
(45) Veröffentlichungstag und Bekanntmachung des Hinweises auf die Patenterteilung: **29.08.2018**
(21) Anmeldenummer: 16167153.2
(22) Anmeldetag: 26.04.2016
(51) Int. Cl.: A61F 2/28, A61F 2/30

(54) **PARTIKULÄRES ALLOPLASTISCHES KNOCHENERSATZMATERIAL UND VERFAHREN ZUR HERSTELLUNG EINES FREI GEFORMTEN PORÖSEN KÖRPERS**
PARTICULATE ALLOPLASTIC BONE REPLACEMENT MATERIAL AND METHOD FOR PRODUCING A FREE-FORMED POROUS BODY
MATERIAU ALLO-PLASTIQUE PARTICULAIRE DE REMPLACEMENT OSSEUX ET PROCÉDÉ DE PRODUCTION D'UN CORPS POREUX DE FORME LIBRE

(30) Priorität: 13.05.2015 DE 102015107600
(43) Veröffentlichungstag der Anmeldung: 16.11.2016
(73) Patentinhaber: Heraeus Medical GmbH, 61273 Wehrheim (DE)
(72) Erfinder: Vogt, Sebastian, 99092 Erfurt (DE)
(74) Vertreter: Schultheiss & Sterzel Patentanwälte PartG mbB

(56) Entgegenhaltungen:
- EP-A1- 1 985 318
- WO-A1-2004/098457
- DE-A1-102012 213 246
- US-A- 5 258 028
- US-A1- 2003 055 511

## Beschreibung

Die Erfindung betrifft ein alloplastisches Knochenersatzmaterial. Die Erfindung betrifft auch ein Verfahren zur Herstellung eines frei geformten Körpers aus einem alloplastischen Knochenersatzmaterial.

Gegenstand der Erfindung ist somit ein alloplastisches Knochenersatzmaterial, das zum Auffüllen und Stabilisieren von Knochenkavitäten bestimmt ist. Ferner wird ein Verfahren zur Herstellung eines frei geformten porösen Körpers vorgeschlagen.

Knochenersatzmaterialien sind seit langem bekannt und werden umfangreich klinisch genutzt (J. M. Rueger: Knochenersatzmittel, Orthopäde 27 (1998) 72-79.). Aus der US 2003/0055511 A1 ist ein autologes partikuläres Knochenersatzmaterial bekannt, bei dem die Partikel Extremitäten aufweisen, wobei die Zwischenräume zwischen den Extremitäten eine Extremität eines benachbarten Partikels aufnehmen können. Die bisher verwendeten Knochenersatzmaterialien sind im Allgemeinen volumenstabil aber nicht formstabil. Eine Ausnahme bildet ein Knochenersatzmaterial, das unter der Bezeichnung "Trabecular metal™" von der Firma Zimmer vertrieben wird und das beispielsweise aus der WO 2013/074 909 A1 bekannt ist. Dieses Material hat eine poröse Struktur, die der Struktur der humanen Spongiosa (Schwammgewebe) nachgebildet ist. Dieses Material besteht aus Tantal und ist in definierten Formen und Größen handelsüblich. Das Material ist im OP nicht in seiner Form und Größe veränderbar. Es kann nicht mit konventionellen Werkzeugen im OP bearbeitet werden. Die jeweilige anatomische Situation des Patienten kann daher nur bedingt berücksichtigt werden. Der medizinische Anwender kann nur versuchen, dass Implantatlager an die vorgegebene Geometrie anzupassen oder ein annähernd passendes Implantat einzusetzend und die bestehenden Lücken mit allogenem Knochenmaterial oder anderem Volumenfüllern zu schließen.

Die Aufgabe der Erfindung besteht also darin, die Nachteile des Stands der Technik zu überwinden. Insbesondere soll ein Knochenersatzmaterial entwickelt werden, das frei geformt werden kann und nach dem Ausformen einen formstabilen porösen Körper bildet, ohne dass chemische Aushärtungsreaktionen, wie beispielsweise radikalische Polymerisationen, notwendig sind. Das Knochenersatzmaterial soll nach der Formung eine offene Porosität besitzen und mechanisch stabil sein. Die Porosität und die Größe der Poren sollen dabei dazu ausreichen und dazu geeignet sein, dass menschlicher Knochen eines Patienten, der mit dem Knochenersatzmaterial behandelt wird, in die Poren einwachsen kann. Es wird ferner angestrebt, dass das Knochenersatzmaterial im geformten Zustand lastragend ist. Weiterhin muss das Knochenersatzmaterial biokompatibel sein, damit es im Körper eines Patienten eingesetzt werden kann.

Die Aufgaben der Erfindung werden gelöst durch ein partikuläres alloplastisches Knochenersatzmaterial aufweisend eine Vielzahl von Partikeln, wobei die Partikel einen Kern und mindestens sechs sich vom Kern erstreckende Stifte aufweisen, wobei die Stifte jeweils mindestens ein Verbindungselement aufweisen und wobei die Stifte elastisch verformbar sind, so dass durch Zusammenpressen mehrerer Partikel die Verbindungselemente verschiedener Partikel miteinander verhaken und/oder rasten und die miteinander verhakten und/oder gerasteten Partikel einen offenporigen Körper aus miteinander verhakten und/oder gerasteten Partikeln bilden.

Bei einem Verhaken greifen Vorsprünge der Verbindungselemente der Stifte der Partikel in Vorsprünge, Greifflächen oder Hinterschnitte von Verbindungselementen an Stiften benachbarter Partikel, so dass anschließend die Stifte noch durch ein weiteres Zusammendrücken der Partikel gegeneinander beweglich sind aber nicht mehr ohne weiteres voneinander getrennt werden können. Bei einem Rasten greifen die Verbindungselemente der Stifte der Partikel derart in Verbindungselemente von Stiften benachbarter Partikel, dass die Partikel nicht mehr ohne weiteres voneinander getrennt werden können, aber auch nicht mehr durch eine weitere Bewegung der Partikel aufeinander zu bewegt werden können, ohne dass dabei eine Verformung der Partikel stattfindet. Die Verbindungselemente können also durch Haken, Nuten, Hinterschnitte, Rastungen und/oder Gegenrastungen beziehungsweise durch Haken, Nuten, Hinterschnitte und/oder Rastelemente gebildet werden.

Die miteinander verhakten und/oder gerasteten Partikel beziehungsweise der dadurch gebildete Volumenkörper ist erfindungsgemäß bevorzugt osteokonduktiv.

Es kann erfindungsgemäß vorgesehen sein, dass die Verbindungselemente Pilze, Haken, Hinterschnitte und/oder Rastelemente sind, vorzugsweise Pilze, Haken, Hinterschnitte, Rastmittel und/oder Gegenrastmittel sind.

Diese Verbindungselemente sind besonders gut zur gegenseitigen Rastung beziehungsweise Verhakung geeignet. Textile Verbindungen, wie beispielsweise Klettverbindungen mit leicht verformbaren Fasern, sind dagegen erfindungsgemäß ungeeignet, da damit keine formstabilen und druckstabilen Körper aufbaubar sind.

Es kann erfindungsgemäß vorgesehen sein, dass der Abstand zwischen den Verbindungselementen und dem Kern der Partikel zwischen 0,25 mm und 2 mm beträgt, bevorzugt zwischen 0,5 mm und 1 mm beträgt.

Es kann bevorzugt auch vorgesehen sein, dass die Partikel sphärisch sind.

Ein Partikel wird im Rahmen der vorliegenden Erfindung als sphärisch angesehen, wenn die Enden der Stifte auf einer Sphäre angeordnet sind. So wären beispielsweise bei einer ikosaedrischen Symmetrie der Partikel die Spitzen der Stifte dieser Partikel alle in einer Kugeloberfläche liegend angeordnet. Sphärische Partikel müssen im Sinne der vorliegenden Erfindung keine geometrisch perfekten Kugeln sein, sondern können auch von der Kugelform abweichen. Bevorzugte sphärische Pulverpartikel weisen eine abgerundete zumindest näherungsweise kugelförmige Form auf und haben ein Verhältnis des längsten Querschnitts zum kürzesten Querschnitt von höchstens 2 zu 1. Im Sinne der vorliegenden Erfindung ist mit einer sphärischen Geometrie also keine streng geometrische beziehungsweise mathematische Kugel gemeint. Die Querschnitte beziehen sich dabei auf innerhalb der Pulverpartikel verlaufende extremale Abmessungen. Besonders bevorzugte sphärische Pulverpartikel können ein Verhältnis des längsten Querschnitts zum kürzesten Querschnitt von höchstens 1,5 zu 1 aufweisen oder ganz besonders bevorzugt kugelförmig sein. Als Durchmesser wird dabei erfindungsgemäß der größte Querschnitt der Pulverpartikel inklusive der nicht elastisch verformten Verbindungselemente angenommen. In jedem Fall weichen die Stifte von einer kugelförmigen Geometrie ab.

Bevorzugte erfindungsgemäße Partikel können sich auch dadurch auszeichnen, dass die Stifte der Partikel sich radial vom Kern erstrecken.

Hierdurch wird erreicht, dass sich die Partikel später besonders leicht miteinander verbinden lassen.

Ferner kann vorgesehen sein, dass die Verbindungselemente an der Mantelfläche der Stifte ausgebildet sind.

Damit kann eine stabile Verbindung der Stifte und damit der Partikel untereinander erreicht werden.

Es wird mit der Erfindung auch vorgeschlagen, dass die ineinander gepressten Partikel irreversibel miteinander verhaken und/oder rasten.

Hierdurch wird sichergestellt, dass sich keine Partikel des fertig geformten Knochenersatzmaterials von dem gebildeten Körper lösen. Dadurch werden Irritationen des behandelten Körpers an der behandelten Stelle verhindert.

Des Weiteren kann vorgesehen sein, dass die Partikel einen maximalen Querschnitt von höchstens 10 mm aufweisen, bevorzugt einen maximalen Querschnitt zwischen 0,5 mm und 10 mm aufweisen, besonders bevorzugt einen maximalen Querschnitt zwischen 1 mm und 4 mm aufweisen.

Die Verbindungselemente gehören zu den Partikeln und tragen somit zum maximalen Querschnitt der Partikel bei. Der maximale Querschnitt ist dabei die Länge der längsten Geraden, die in der geometrischen Form des Partikels angeordnet werden kann. Damit wird erreicht, dass ausreichend feine Strukturen erzeugt werden können. Gleichzeitig sollen die Partikel aber nicht zu aufwendig und kostspielig in der Herstellung sein.

Ferner kann vorgesehen sein, dass die Partikel mit einem generativen 3D-Druckverfahren hergestellt werden.

Hierdurch lassen sich die Partikel und damit das Knochenersatzmaterial kostengünstig herstellen.

Gemäß einer Weiterbildung der vorliegenden Erfindung kann vorgesehen sein, dass zumindest eines der zumindest einen Verbindungselemente pro Stift stumpf-kegelförmig ausgebildet ist, wobei die Längsachsen der Stifte die Längsachsen der Kegel bilden und wobei der Mantel der Kegel nach der zum Kern abgewandten Außenseite gerichtet ist.

Hierdurch wird eine besonders stabile Verbindbarkeit der Partikel über die als Kegelstümpfe geformten Verbindungselemente erreicht. Zudem wird durch diese Formgebung nach der Implantation des Knochenersatzmaterials verhindert, dass umgebendes Weich- und Knochengewebe verletzt wird.

Bevorzugt kann auch vorgesehen sein, dass zumindest eines der zumindest einen Verbindungselemente pro Stift als Haken oder als Pilzkopf ausgebildet ist.

Die Haken und/oder die Pilzköpfe sorgen für eine stabile und unlösbare Verbindung der Partikel untereinander. Wenn die Verbindungselemente als Pilzköpfe geformt sind, können diese zum Beispiel einen in Richtung Kern ausgebildeten Kragen am Pilzkopfrand besitzen, so dass in diesem Hinterschnitt hakenförmige Verbindungselemente von anderen Partikeln eingreifen können, wodurch eine irreversible, nicht lösbare Verhakung oder Rastung zwischen den Partikeln entsteht. Es ist auch möglich und erfindungsgemäß bevorzugt, dass jeweils ein Partikel verschiedene Verbindungselemente enthält oder verschiedene Stifte mit unterschiedlichen Verbindungselementen aufweist. So kann ein Partikel zum Beispiel Haken und Pilzköpfe als Verbindungselemente gleichzeitig besitzen sowohl am gleichen Stift als auch an unterschiedlichen Stiften.

In einer bevorzugten Ausführungsform sind die Verbindungselemente als Pilzköpfe ausgebildet. In einer besonders bevorzugten Ausführungsform sind die Pilzköpfe so gestaltet, dass die Pilzköpfe an der dem Kern zugewandten Seite einen kegelförmigen Hinterschnitt aufweisen. Dadurch können hakenförmige Rastelemente mit diesen Pilzköpfen irreversibel und unlösbar verhakt werden. Bei geeigneter zueinander passender Form der Hinterschnitte zu den Pilzköpfen kann auch ein weiteres Vortreiben der Pilzköpfe verhindert werden, so dass die Pilzköpfe mit den Hinterschnitten rasten.

Mit einer Weiterbildung der vorliegenden Erfindung wird auch vorgeschlagen, dass die Stifte zwischen dem Kern und zumindest einem der zumindest einen Verbindungselemente eine umlaufende Nut als zusätzliches Verbindungselement enthalten, in welche Verbindungselemente anderer Partikel einhaken oder einrasten können, bevorzugt derart einrasten, dass keine weitere Bewegung der Verbindungselemente entlang der Stifte möglich ist.

Auch hierdurch wird eine besonders stabile Verbindung der Partikel ermöglicht. Vorteilhaft ist dabei ferner, dass dadurch definierte und freibleibende Hohlräume nach der Rastung der Partikel in dem so aus dem Knochenersatzmaterial geformten Körper erreicht werden. Es wird nämlich ein weiteres verschließen der offenporigen Struktur durch ein weiteres vorantreiben der Stifte zwischen den Stiften eines benachbarten Partikels verhindert und so die Poren offen gehalten.

Gemäß einer erfindungsgemäßen Variante kann vorgesehen sein, dass zumindest zwei Verbindungselemente hintereinander auf der Mantelfläche der Stifte angeordnet sind, besonders bevorzugt zumindest drei Verbindungselemente hintereinander auf der Mantelfläche der Stifte angeordnet sind.

Hierdurch wird es möglich, die Partikel mit unterschiedlichen Abständen zueinander zu verhaken und/oder zu rasten. Dadurch wird eine höhere Flexibilität beim Formen des Knochenersatzmaterials erreicht.

Es kann erfindungsgemäß ferner vorgesehen sein, dass die Partikel kugelförmig, bohnenförmig, quaderförmig, würfelförmig und/oder polyedrisch ausgebildet sind, vorzugsweise mit kubischer, oktaedrischer, dodekaedrischer, ikosaedrischer und/oder trikontaedrischer Symmetrie ausgebildet sind.

Durch diese Symmetrien können stabile und beliebig geformte Körper aus dem Knochenersatzmaterial geformt werden. Die Verbindungsmittel können dabei eine Form aufweisen, die die Symmetrie der Partikel brechen. Dennoch wird den Partikeln die entsprechende Symmetrie zugeordnet.

Bevorzugte erfindungsgemäße Knochenersatzmaterialien können sich auch dadurch auszeichnen, dass die Partikel einen maximalen Querschnitt von größer 1 mm, bevorzugt einen maximalen Querschnitt von größer 2 mm und besonders bevorzugt einen maximalen Querschnitt von größer 3 mm aufweisen.

Hierdurch können die Partikel ohne Mikrostrukturierung kostengünstig gefertigt werden.

Bevorzugt kann des Weiteren vorgesehen sein, dass die Partikel aus biokompatiblem Kunststoff, Edelstahl, Titan, einer Titanlegierung, Tantal, einer Tantallegierung oder aus Kompositen dieser Materialien gebildet sind.

Diese Materialien sind für medizinische Zwecke besonders gut einsetzbar und mit diesen Materialien lassen sich auch die geeigneten elastischen Eigenschaften für die Stifte einstellen. Aus Metall oder Metalllegierungen bestehende Partikel können erfindungsgemäß bevorzugt durch selektives Lasersintern oder auch durch Schmelzen mit Elektronenstrahlen hergestellt werden, bevorzugt mit einem 3D-Druckverfahren.

Der biokompatible Kunststoff kann biodegradierbar sein. Dazu können Polylactide, Polylglycolide, Polycaprolactone und Polyester verwendet werden, die aus unterschiedlichen α-Hydroxycarbonsäuren gebildet sind. Als nicht biodegradierbare Kunststoffe kommen Polyamide, Polyimide, Polyetherketon und Polysulfon in Betracht. Partikel, insbesondere sphärische Partikel, aus diesen nicht biodegradierbaren und biodegradierbaren Kunststoffen können durch selektives Lasersintern hergestellt werden.

Gemäß einer Weiterbildung der vorliegenden Erfindung kann vorgesehen sein, dass benachbarte Stifte eines Partikels einen derartigen Abstand zueinander aufweisen, dass nach einer elastischer Deformation aufgrund einer Verhakung und/oder Rastung mit einem Verbindungselement eines anderen Partikels die Stifte des Partikels mindestens zwei Verhakungen und/oder Rastungen mit zwei weiteren Partikeln ermöglichen, bevorzugt mindestens drei Verhakungen und/oder Rastungen mit drei weiteren Partikeln ermöglichen, ganz besonders bevorzugt mehr als drei Verhakungen und/oder Rastungen mit mehr als drei weiteren Partikeln ermöglichen.

Durch eine mehrfache Verhakungen und/oder Rastung der Partikel kann ein besonders stabiler Körper aus dem Knochenersatzmaterial geformt werden.

Bevorzugt kann auch vorgesehen sein, dass die Partikel in einer wässrigen oder nichtwässrigen Lösung von biokompatiblen Polymeren und/oder Oligomeren suspendiert sind und mit der Lösung eine pastöse Masse bilden.

Hierdurch kann das Knochenersatzmaterial besonders einfach verarbeitet werden. Zudem kann die Lösung weitere zur Behandlung hilfreiche Substanzen enthalten. Als biokompatible Polymere können Hyaluronsäure, Hydroxyethylstärke, Hydroxyethylcellulose, Methylcellulose, Carboxymethylcellulose, oxidierte Cellulose und Cellulosesulfat verwendet werden. Weiterhin ist auch die Verwendung von Gelatine als biokompatibles Polymer möglich. Daneben ist auch die Verwendung von in Wasser löslichen Polymethacrylsäurederivaten möglich. Auch kann Polyvinylalkohol und Polyvinylpyrrolidon Verwendung finden.

Alternativ kann vorgesehen sein, dass die Partikel in einer bei Raumtemperatur hydrophoben, niedermolekularen Flüssigkeit suspendiert sind und mit der Flüssigkeit eine pastöse Masse bilden.

Durch diese beiden Alternativen kann das Knochenersatzmaterial besonders einfach verarbeitet werden. Zudem kann die Lösung beziehungsweise die Flüssigkeit weitere zur Behandlung hilfreiche Substanzen enthalten. Als hydrophobe, niedermolekulare Flüssigkeiten kommen Polyethylenglykol mit einer Molmasse kleiner 1.000 g/mol, Fettsäureester des Glycerins, Methyl-, Ethyl- und Isopropylester von Fettsäuren in Betracht.

Des Weiteren wird vorgeschlagen, dass die Partikel mit anorganischem oder organischem partikulärem Knochenersatzmaterial und/oder autologer oder auch allogener Spongiosa vermischt sind.

Hiermit kann die Knochenheilung und die Verbindung des Knochenersatzmaterials mit dem Knochen beschleunigt werden.

Mit einer Weiterbildung wird auch vorgeschlagen, dass die Partikel in einer biokompatiblen Flüssigkeit suspendiert sind, die einen oder mehrere pharmazeutischen Wirkstoffe enthält, wobei der oder die pharmazeutischen Wirkstoffe in der Flüssigkeit suspendiert und/oder gelöst sind.

Hiermit wird eine pharmakologische Wirkung des Knochenersatzwerkstoffs erreicht, die zur Heilung des mit dem Knochenersatzmaterial behandelten Patienten beiträgt. Aus der Gruppe der Antibiotika als pharmazeutische Wirkstoffe sind besonders Gentamicin, Tobramycin, Amikacin, Vancomycin, Teicoplanin, Clindamycin und Daptomycin bevorzugt.

Bevorzugt kann ferner vorgesehen sein, dass die Partikel mit einem oder mehreren pharmazeutischen Wirkstoffen aus den Gruppen der Antibiotika, der Bisphosphonate, der Steroide, der nichtsteroidalen Entzündungshemmer, der Wachstumsfaktoren und der Cytostatika beschichtet sind.

Auch hiermit wird eine pharmakologische Wirkung des Knochenersatzwerkstoffs erreicht, die zur Heilung des mit dem Knochenersatzmaterial behandelten Patienten beiträgt. Aus der Gruppe der Antibiotika sind besonders Gentamicin, Tobramycin, Amikacin, Vancomycin, Teicoplanin, Clindamycin und Daptomycin bevorzugt.

Besonders bevorzugte Ausführungsformen können sich dadurch auszeichnen, dass die Partikel mindestens vierzehn sich vom Kern erstreckende Stifte aufweisen, bevorzugt mindestens zwanzig sich vom Kern erstreckende Stifte aufweisen, besonders bevorzugt zwischen zwanzig und fünfzig sich vom Kern erstreckende Stifte aufweisen, ganz besonders bevorzugt zwischen dreißig und vierzig sich vom Kern erstreckende Stifte aufweisen.

Mit höherer Anzahl sich vom Kern erstreckender Stifte insbesondere sich radial vom Kern erstreckender Stifte steigt die Anzahl der Zwischenräume und damit der Verbindungsmöglichkeiten zwischen den Partikeln.

Des Weiteren kann vorgesehen sein, dass die Poren des aus den Partikeln gebildeten offenporigen Körpers interkonnektierend und osteokonduktiv sind, wobei vorzugsweise die Poren einen freien Querschnitt zwischen 0,1 mm und 1 mm aufweisen, besonders bevorzugt zwischen 0,25 mm und 0,9 mm.

Hiermit wird sichergestellt, dass der Knochen gut mit den Poren des aus dem Knochenersatzmaterial gebildeten Körpers verwachsen kann.

Gemäß einer besonders bevorzugten Ausführungsform der vorliegenden Erfindung kann vorgesehen sein, dass das Knochenersatzmaterial neben den Partikeln zusätzlich zumindest eine Platte aufweist, wobei die zumindest eine Platte ein Flächengebilde aufweist und eine Mehrzahl von sich aus dem Flächengebilde der zumindest einen Platte heraus erstreckende Stifte aufweist, wobei die Stifte jeweils mindestens ein Verbindungselement aufweisen, das analog der Verbindungselemente der Partikel ausgebildet ist, so dass die Partikel mit der zumindest einen Platte durch Aufeinanderpressen die Verbindungselemente verschiedener Platten und Partikel miteinander verhaken und/oder rasten und die miteinander verhakten und/oder gerasteten Platten und Partikel einen offenporigen Körper aus miteinander verhakten und/oder gerasteten Platten und Partikeln bilden.

Dadurch wird ein noch variabler nutzbares Knochenersatzmaterial erzielt, das sowohl frei geformt, als auch zur Überbrückung von Spalten und Kavitäten genutzt werden kann.

Die der vorliegenden Erfindung zugrundeliegenden Aufgaben werden auch gelöst durch ein Verfahren zum Formen eines Körpers aus einem erfindungsgemäßen partikulären alloplastischen Knochenersatzmaterial, bei dem die Partikel gegeneinander gedrückt werden, wobei die Partikel dadurch miteinander verhaken und/oder rasten und einen offenporigen Körper ausbilden.

Dabei kann vorgesehen sein, dass die Partikel in Kontakt miteinander gebracht werden, bevor sie gegeneinander gedrückt werden.

Ferner kann vorgesehen sein, dass die Partikel mit einem porösen Volumenkörper eines zweiten Knochenersatzmaterials verbunden werden, indem die Verbindungselemente mit den Poren des zweiten Knochenersatzmaterials rasten und/oder verhaken, und/oder die Partikel mit einem flächigen dritten Knochenersatzmaterial verbunden werden, das eine Vielzahl von Stiften mit Verbindungelementen aufweist, wobei bevorzugt die Stifte und die Verbindungselemente des flächigen dritten Knochenersatzmaterials die Merkmale der Stifte und Verbindungselemente der Partikel des erfindungsgemäßen Knochenersatzmaterials aufweisen.

Der poröse Volumenkörper des zweiten Knochenersatzmaterials kann beispielsweise ein Trabecular metal™ von der Firma Zimmer sein.

Die der vorliegenden Erfindung zugrundeliegenden Aufgaben werden schließlich auch gelöst durch die Verwendung des erfindungsgemäßen alloplastischen Knochenersatzmaterials als Implantatmaterial in der Unfallchirurgie, Orthopädie oder in der Veterinärmedizin.

Bei Druckeinwirkung auf die miteinander in Berührung stehenden (bevorzugt sphärischen) Partikel des Knochenersatzmaterials bilden diese erfindungsgemäß einen mechanisch stabilen Verbund aus.

Der Erfindung liegt die überraschende Erkenntnis zugrunde, dass mechanisch rastende beziehungsweise verhakende Partikel als alloplastisches Knochenersatzmaterial verwenden werden können. Dabei können die Partikel in eine gewünschte Form gebracht werden und durch Druck miteinander rasten und dabei miteinander verbunden werden. Durch eine geeignete Form der Partikel wird dabei ein poröses Knochenersatzmaterial gebildet, das für die medizinische Anwendung mechanisch stabil genug ist. Der Knochen kann in die Poren des durch Druck verbundenen Knochenersatzmaterials einwachsen und sich so mit dem Knochenersatzmaterial dauerhaft verbinden.

Überraschend wurde gefunden, dass das erfindungsgemäße Knochenersatzmaterial in Form von Partikeln in beliebig geformte Kavitäten eingebracht werden kann und durch einfaches Komprimieren per Hand oder mit Hilfe eines Stößels zu einem porösen, aber homogenen Körper unter Verhakung der einzelnen sphärischen Partikel ausgehärtet werden kann. Damit wird ein Vorteil gegenüber den bisherigen Knochenersatzmaterial "Trabecular Metal™" erreicht, dessen Form und Größe nicht frei vom medizinischen Anwender bestimmt werden kann. Es ist damit möglich, beliebig geformte Knochenkavitäten mit einem in situ aushärtenden beziehungsweise festwerdenden Knochenersatzmaterial aufzufüllen, dass ohne chemische Reaktionen, wie zum Beispiel radikalische Polymerisationen, notwendig sind. Eine Aushärtung des erfindungsgemäßen Knochenersatzmaterials ist durch einfaches Komprimieren von aneinander liegenden sphärischen Partikeln möglich.

Mechanisch verhakende Systeme nach dem Bauprinzip von Kletten sind seit mehreren Jahrzehnten bekannt. Erstmalig wurde das Prinzip des Klettverschlusses von de Mestral in CH 295 638 A beschrieben. Dieses Prinzip wurde weiterentwickelt und findet bei unterschiedlichsten, reversibel schließenden Klettverschlüssen Verwendung. Beispielhaft für die Weiterentwicklung sind die Schriften DE 1 610 318 A1, DE 1 625 396 A1, US 5 077 870 A und US 4 290 174 A.

Eine interessante Weiterentwicklung erfolgte später, bei der reversibel verklettende Stahlbandsysteme für mechanisch hochbelastbare Anwendungen und für Anwendungen bei hohen Temperaturen entwickelt wurden (DE 10 2004 048 464 A1, DE 10 2006 015 100 A1, DE 10 2006 015 145 A1, DE 10 2006 015 148 A1).

Es wurde im Rahmen der vorliegenden Erfindung überraschend gefunden, dass solche Systeme beziehungsweise derartige Funktionsprinzipien für Knochenersatzmaterialien einsetzbar beziehungsweise auf Knochenersatzmaterialien übertragbar sind. Dabei ist es für die Knochenersatzmaterialien von Vorteil, dass derartige Verbindungen nicht dicht abschließen, sondern Zwischenräume als offenporige Struktur verbleiben. Die sich dadurch im Volumenkörper bildenden interkonnektierenden Poren können mit dem Knochen verwachsen und damit eine stabile Verbindung zwischen dem Knochen und dem Knochenersatzmaterial erzeugen. Hierzu muss sichergestellt werden, dass die Poren in dem Knochenersatzmaterial einen ausreichenden freien Querschnitt aufweisen. Die Poren werden als osteokonduktiv bezeichnet, wenn der Knochen in die Poren einwachsen kann und sich somit mit dem aus dem Knochenersatzmaterial gebildeten Körper verbindet.

Ein beispielhaftes und erfindungsgemäß besonders bevorzugtes Ausführungsbeispiel der vorliegenden Erfindung ist ein partikuläres alloplastisches Knochenersatzmaterial aufweisend eine Vielzahl sphärischer Partikel, wobei die Partikel aus einem Kern und mindestens sechs vom Kern radial ausgehenden Stiften gebildet sind, wobei an der Mantelfläche jedes Stiftes mindestens ein Rastelelement ausgebildet ist und wobei die Stifte mit den Rastelementen aus mindestens einem elastisch verformbaren Material gebildet sind. Der Aufbau der Partikel wird dabei derart gestaltet und dass durch Zusammenpressen der sphärischen Partikel sich kontaktierende sphärische Partikel irreversibel miteinander rasten oder verhaken und einen porösen Körper aus miteinander verhakten oder gerasteten sphärischen Partikeln bilden.

Im Folgenden werden weitere Ausführungsbeispiele der Erfindung anhand von vierzehn schematisch dargestellten Figuren erläutert, ohne jedoch dabei die Erfindung zu beschränken. Dabei zeigt:
Figur 1: eine schematische perspektivische Ansicht eines Partikels eines erfindungsgemäßen Knochenersatzmaterials;
Figur 2: eine schematische Querschnittansicht des Partikels nach Figur 1;
Figur 3: eine schematische perspektivische Ansicht eines erfindungsgemäßen Knochenersatzmaterials aus mehreren Partikeln nach den Figuren 1 und 2, die miteinander verbunden sind;
Figur 4: eine schematische Querschnittansicht (links) und schematische perspektivische Ansicht (rechts) zweier Partikel eines zweiten alternativen erfindungsgemäßen Knochenersatzmaterials;
Figur 5: eine schematische perspektivische Ansicht dreier miteinander verbundener Partikel des zweiten erfindungsgemäßen Knochenersatzmaterials nach Figur 4;
Figur 6: eine schematische geschnittene Ansicht eines Partikels eines dritten alternativen erfindungsgemäßen Knochenersatzmaterials;
Figur 7: eine schematische perspektivische Ansicht eines Ausschnitts des Partikels nach Figur 6;
Figur 8: eine schematische Querschnittansicht eines Ausschnitts des Partikels nach Figur 6;
Figur 9: eine schematische perspektivische Ansicht von vier Partikeln nach Figur 6, von denen drei miteinander verbunden sind;
Figur 10: eine schematische Querschnittansicht (rechts) und schematische perspektivische Ansicht (links) zweier Partikel eines vierten alternativen erfindungsgemäßen Knochenersatzmaterials;
Figur 11: eine schematische Querschnittansicht zweier miteinander verbundener Partikel des vierten erfindungsgemäßen Knochenersatzmaterials nach Figur 10;
Figur 12: eine schematische perspektivische Ansicht dreier miteinander verbundener Partikel des vierten erfindungsgemäßen Knochenersatzmaterials nach Figur 10 und 11 und mehrere Platten eines Knochenersatzmaterials, die mit den Partikeln verbunden werden können;
Figur 13: eine schematische perspektivische Ansicht zweier verbundener Partikel eines fünften alternativen erfindungsgemäßen Knochenersatzmaterials; und
Figur 14: eine schematische Querschnittansicht der verbundenen Partikel nach Figur 13.

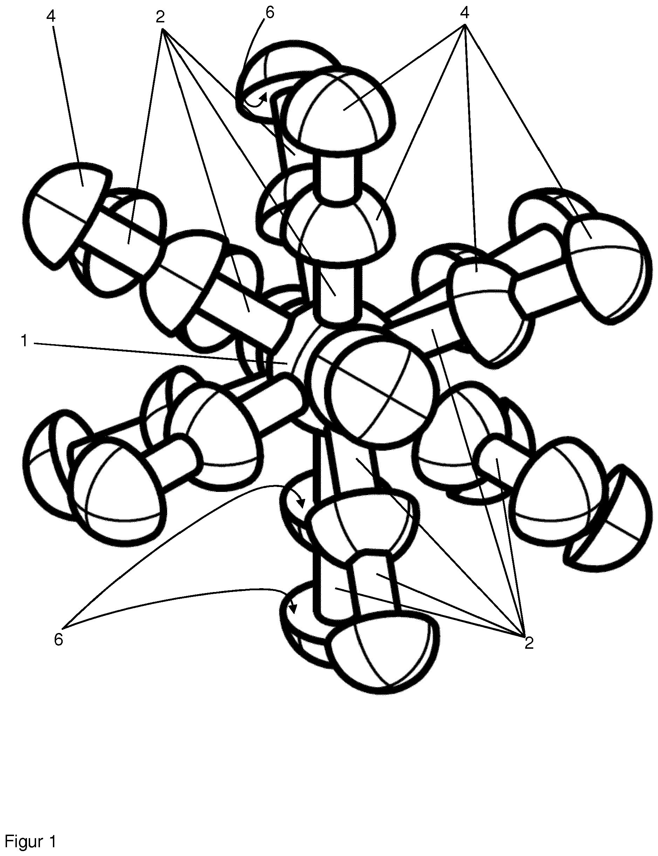

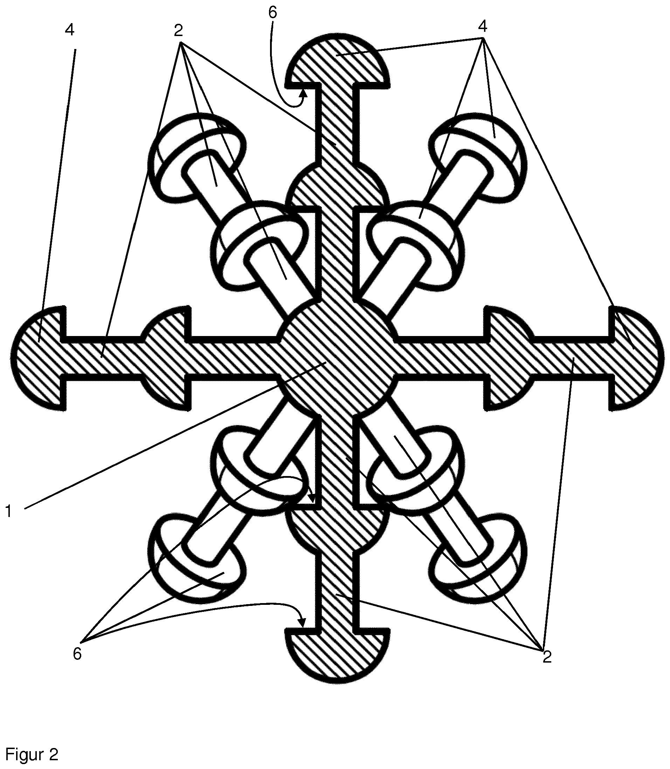

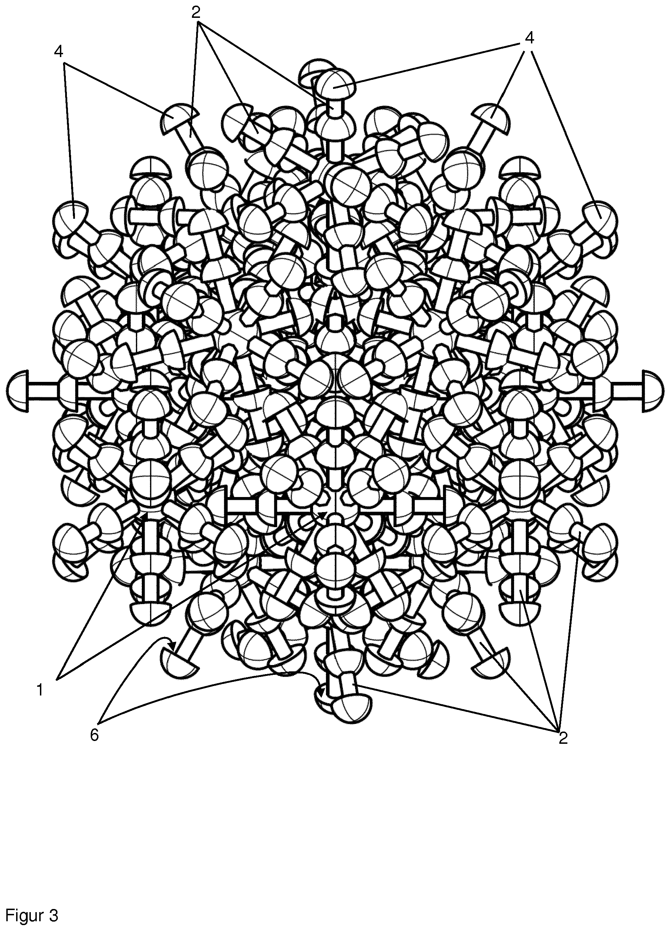

Die Figuren 1 und 2 zeigen eine schematische perspektivische Ansicht eines Partikels eines erfindungsgemäßen Knochenersatzmaterials und eine schematische Querschnittansicht eines solchen Partikels. Figur 3 zeigt dazu eine schematische perspektivische Ansicht eines erfindungsgemäßen Knochenersatzmaterials aus mehreren Partikeln, die in den Figuren 1 und 2 gezeigt sind und die miteinander zu einem offenporigen Körper verbunden sind. Die Partikel bestehen aus einem elastischen biokompatiblem Kunststoff können aber auch aus Edelstahl, Titan, einer Titanlegierung, Tantal, einer Tantallegierung oder aus Kompositen dieser Materialien gefertigt sein. Die Partikel werden mit einem CAM-Verfahren (CAM - Computer-Aided Manufacturing, Deutsch: rechner-unterstützte Fertigung) beziehungsweise einem 3D-Druckverfahren hergestellt, beispielsweise mit selektivem Laserschmelzen SLM (Selective Laser Melting). Andere Rapid-Prototyping-Verfahren beziehungsweise computergestützte generative Herstellungsverfahren können auch zur Herstellung der Partikel verwendet werden, wie beispielsweise Fused Layer Modeling/Manufacturing (FLM), Fused Deposition Modeling (FDM), Laminated Object Modelling (LOM) von Kunststofffolien, Layer Laminated Manufacturing (LLM) von Kunststofffolien, Elektronenstrahlschmelzen (EBM) von Kunststoffen oder Metallen, Multi Jet Modeling (MJM) von Kunststoffen, Selektives Lasersintern (SLS) von Kunststoffen oder Metallen, Stereolithografie (STL oder SLA) von Kunststoffen, Schleif- oder Mehr-Achs-Fräsverfahren oder Digital Light Processing (DLP) von photopolymerisierbaren flüssigen Kunststoffen.

Die Partikel sind aufgebaut aus einem Kern 1, der in dem geometrischen Zentrum des Partikels angeordnet ist, sowie vierzehn Stiften 2, die sich in verschiedene Richtungen radial vom Kern 1 weg erstrecken. An den Stiften 2 sind jeweils zwei Pilze 4 (beziehungsweise Pilzköpfe 4) als Verbindungselemente an den ansonsten zylindrischen Stiften 2 angeordnet. Die Pilze 4 sind nach außen (vom Kern 1 weg) abgerundet und bilden Kugelabschnitte. Es sind aber auch andere Abrundungen, wie beispielsweise Ellipsenabschnitte möglich. An der zum Kern 1 hin ausgerichteten Seite bilden die Pilze 4 eine ebene Greiffläche 6, die zum Verhaken mit anderen Pilzen 4 eingreifender Partikel geeignet sind. Bevorzugt haben die Pilze 4 einen etwas größeren Durchmesser als den in den Figuren 1 bis 3 gezeigten, damit sie leichter ineinandergreifen und verhaken können.

Um ein erfindungsgemäßes Knochenersatzmaterial zu bilden, liegen die Partikel bevorzugt aneinander anliegend aber nicht miteinander verhakt vor, so dass also die Pilze 4 der Stifte 2 noch nicht ineinander greifen. Zudem können die Partikel mit einer Flüssigkeit gemischt als Schlämmung vorliegen. In der Flüssigkeit ist bevorzugt zumindest eine pharmazeutisch wirksame Substanz enthalten, die zur Bekämpfung der Infektion oder zum Anregen des Knochenwachstums geeignet ist. Alternativ oder zusätzlich können die Partikel mit einer solchen pharmazeutisch wirksamen Substanz beschichtet sein.

Das Knochenersatzmaterial kann geformt werden, indem die Partikel ineinander gedrückt werden. Dadurch verhaken oder rasten die Partikel miteinander und das Knochenersatzmaterial wird in der gewünschten Form verfestigt. Die Partikel verbinden sich dabei untereinander in der Art, dass freie Zwischenräume zwischen den miteinander verbundenen Partikeln verbleiben, so dass der aus den Partikeln geformte Volumenköper offenporig ist. Die Partikel haben einen Durchmesser von etwa 5 mm, so dass die verbleibenden Poren einen freien Querschnitt im Bereich von etwa 0,5 mm aufweisen. Dieser Querschnitt reicht aus, damit sich in den Poren Knochenmaterial ausbilden beziehungsweise einwachsen kann. Der Körper mit seinen offenen Poren kann also als osteokonduktiv bezeichnet werden. Der aus den Partikeln geformte Volumenkörper ist daher als Knochenersatzmaterial gut geeignet.

Die Partikel sollten fest ineinander gedrückt werden, so dass der Volumenkörper formstabil ist. Die Partikel rasten dabei ineinander dadurch, dass die Pilze 4 die Stifte 2 verbundener Partikel elastisch verformen und durch die elastische Rückstellkraft der Stifte 2 die Pilze 4 benachbarter Partikel zum Kern 1 hin gezogen werden. Es ist auch möglich, dass die Kanten der Pilze 4 die Stifte 2 oder die Pilze 4 benachbarter Partikel in geringem Umfang plastisch verformen und dadurch eine Rastung der Partikel miteinander erfolgt.

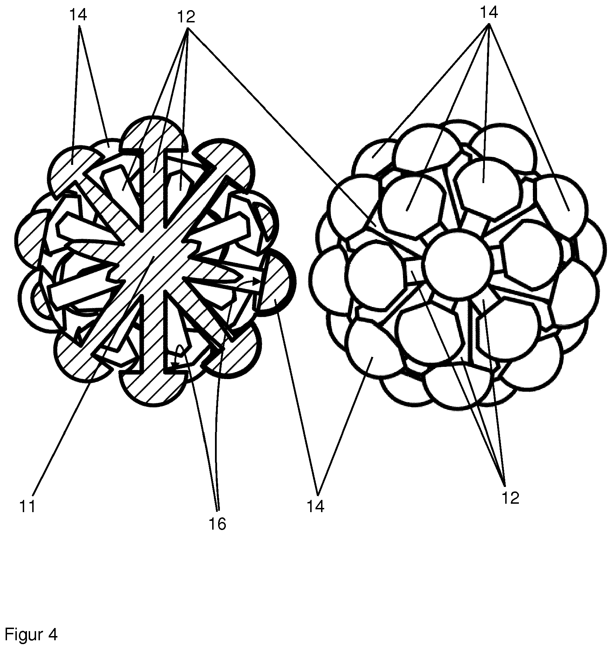

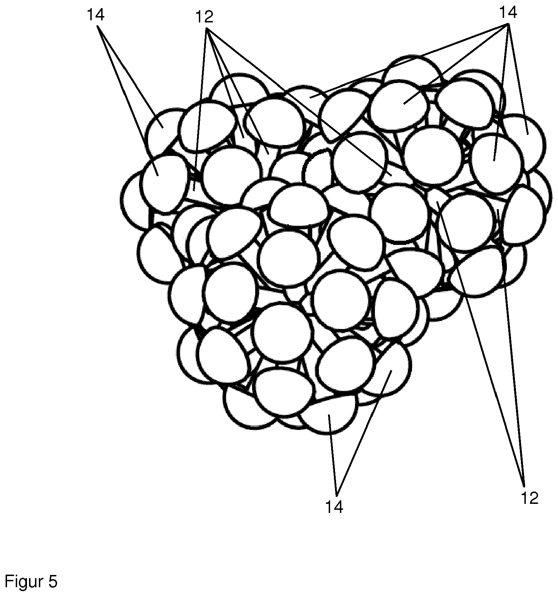

Figur 4 zeigt eine schematische Querschnittansicht (links) und schematische perspektivische Ansicht (rechts) zweier Partikel eines zweiten alternativen erfindungsgemäßen Knochenersatzmaterials und Figur 5 eine schematische perspektivische Ansicht dreier miteinander verbundener Partikel des zweiten erfindungsgemäßen Knochenersatzmaterials nach Figur 4 und die miteinander zu einem offenporigen Körper verbunden sind. Die Partikel bestehen aus Edelstahl, Titan, einer Titanlegierung, Tantal und/oder einer Tantallegierung, können aber auch oder aus Kompositen dieser Materialien oder einem biokompatiblem Kunststoff gefertigt sein. Die Partikel werden mit einem CAM-Verfahren beziehungsweise einem 3D-Druckverfahren hergestellt, beispielsweise mit selektivem Elektronenstrahlschmelzen. Andere Rapid-Prototyping-Verfahren können auch zur Herstellung der Partikel verwendet werden.

Die Partikel sind aufgebaut aus einem Kern 11, der in dem geometrischen Zentrum des Partikels angeordnet ist, sowie zweiunddreißig Stiften 12, die sich in verschiedene Richtungen radial vom Kern 11 weg erstrecken. An den Enden der Stifte 12 ist jeweils ein Pilz 14 als Verbindungselemente an den ansonsten zylindrischen Stiften 12 angeordnet. Die Pilze 14 sind nach außen (vom Kern 11 weg) abgerundet und bilden Kugelabschnitte. Es sind aber auch andere Abrundungen, wie beispielsweise Ellipsenabschnitte möglich. An der zum Kern 11 hin ausgerichteten Seite bilden die Pilze 14 eine ebene Greiffläche 16, die zum Verhaken mit anderen Pilzen 14 eingreifender Partikel geeignet sind.

Um ein erfindungsgemäßes Knochenersatzmaterial zu bilden, liegen die Partikel bevorzugt aneinander anliegend aber nicht miteinander verhakt vor, so dass also die Pilze 14 der Stifte 12 noch nicht ineinander greifen. Zudem können die Partikel mit einer Flüssigkeit gemischt als Schlämmung vorliegen. In der Flüssigkeit ist bevorzugt zumindest eine pharmazeutisch wirksame Substanz enthalten, die zur Bekämpfung der Infektion oder zum Anregen des Knochenwachstums geeignet ist. Alternativ oder zusätzlich können die Partikel mit einer solchen pharmazeutisch wirksamen Substanz beschichtet sein.

Das Knochenersatzmaterial kann geformt werden, indem die Partikel ineinander gedrückt werden. Dadurch verhaken oder rasten die Partikel miteinander und das Knochenersatzmaterial wird in der gewünschten Form verfestigt. Die Partikel verbinden sich dabei untereinander in der Art, dass freie Zwischenräume zwischen den miteinander verbundenen Partikeln verbleiben, so dass der aus den Partikeln geformte Volumenköper offenporig ist. Die Partikel haben einen Durchmesser von etwa 3 mm, so dass die verbleibenden Poren einen freien Querschnitt im Bereich von etwa 0,3 mm aufweisen. Dieser Querschnitt reicht noch aus, damit sich in den Poren Knochenmaterial ausbilden beziehungsweise einwachsen kann. Der Körper mit seinen offenen Poren kann also als osteokonduktiv bezeichnet werden. Der aus den Partikeln geformte Volumenkörper ist daher als Knochenersatzmaterial gut geeignet.

Die Partikel sollten fest ineinander gedrückt werden, so dass der Volumenkörper formstabil ist. Die Partikel rasten dabei ineinander dadurch, dass die Pilze 14 die Stifte 12 verbundener Partikel elastisch verformen und durch die elastische Rückstellkraft der Stifte 12 die Pilze 14 benachbarter Partikel zum Kern 11 hin gezogen werden. Es ist auch möglich, dass die Kanten der Pilze 14 die Stifte 12 oder die Pilze 14 benachbarter Partikel in geringem Umfang plastisch verformen und dadurch eine Rastung der Partikel miteinander erfolgt.

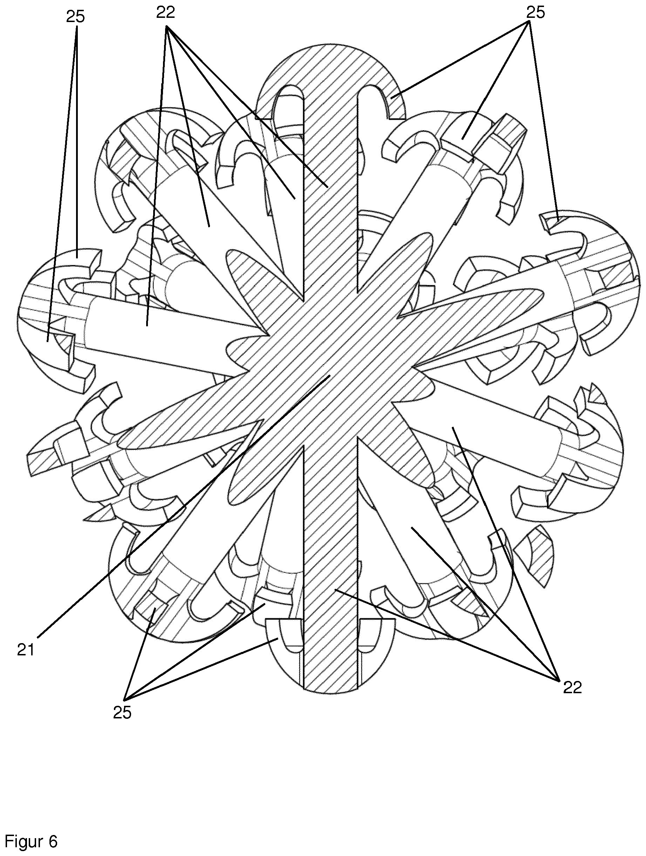

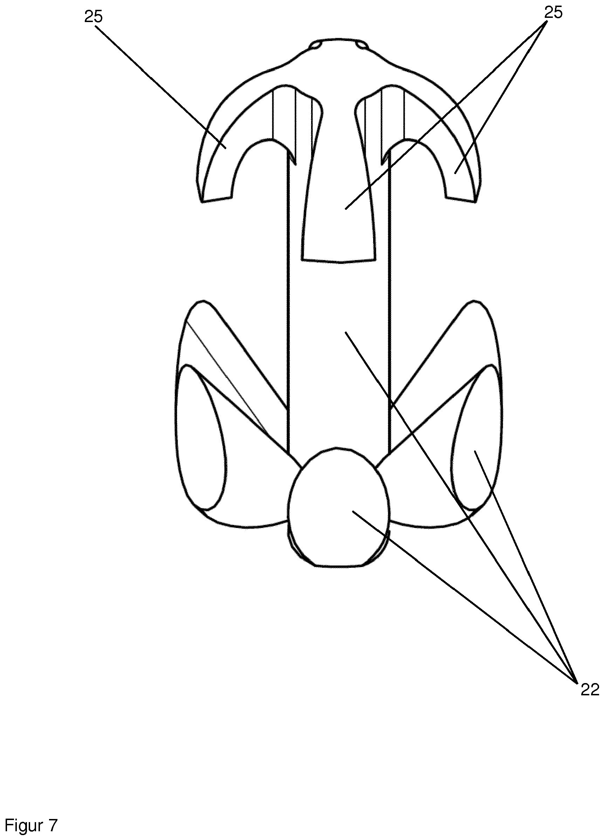

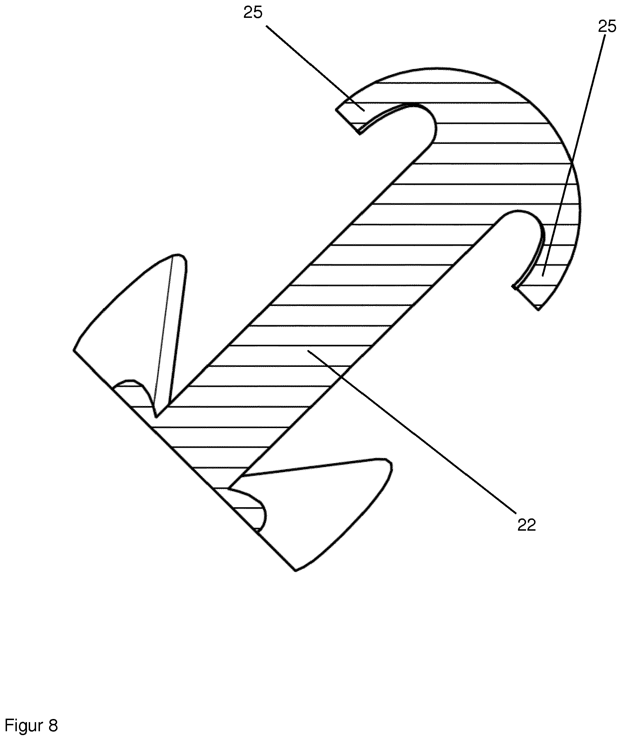

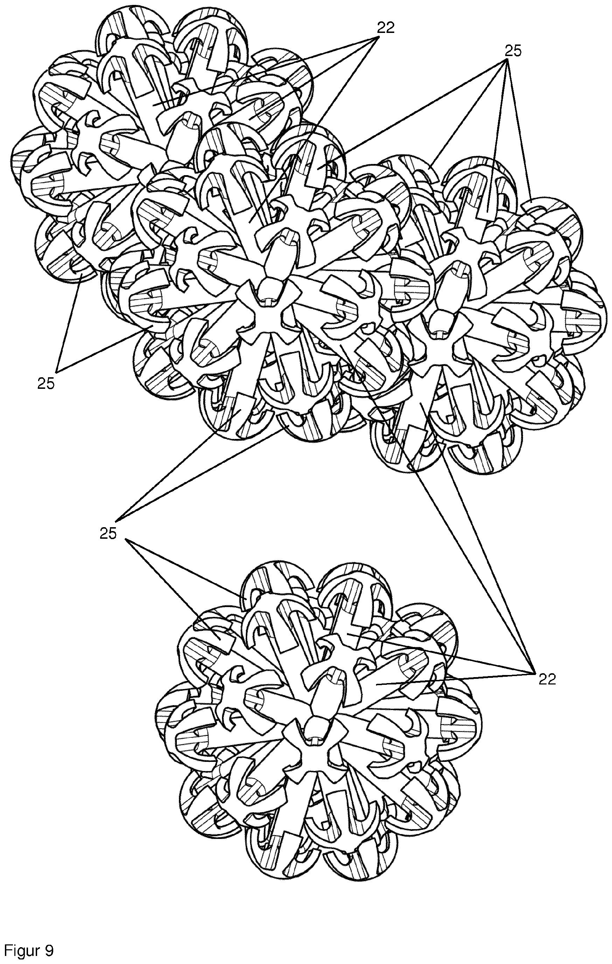

Figur 6 zeigt eine schematische geschnittene Ansicht eines Partikels eines dritten alternativen erfindungsgemäßen Knochenersatzmaterials, Figur 7 eine schematische perspektivische Ansicht eines Ausschnitts des Partikels nach Figur 6, Figur 8 eine schematische Querschnittansicht eines Ausschnitts des Partikels nach Figur 6 und Figur 9 eine schematische perspektivische Ansicht von vier Partikeln nach Figur 6, von denen drei miteinander zu einem offenporigen Körper verbunden sind. Die Partikel bestehen aus Tantal oder einer Tantallegierung, können aber auch aus anderen biokompatiblen Metallen oder biokompatiblen Metalllegierungen oder aus einem biokompatiblen Kunststoff gefertigt sein. Die Partikel werden mit einem CAM-Verfahren beziehungsweise einem 3D-Druckverfahren hergestellt, beispielsweise mit selektivem Elektronenstrahlschmelzen. Andere Rapid-Prototyping-Verfahren können auch zur Herstellung der Partikel verwendet werden.

Die Partikel sind aufgebaut aus einem Kern 21, der in dem geometrischen Zentrum des Partikels angeordnet ist, sowie zweiunddreißig Stiften 22, die sich in verschiedene Richtungen radial vom Kern 21 weg erstrecken. An den Enden der Stifte 22 ist eine Gruppe von vier Haken 25 als Verbindungselemente an den ansonsten zylindrischen Stiften 22 angeordnet. Die Haken 25 sind nach außen (vom Kern 21 weg) kugelförmig abgerundet geformt. Es sind auch andere Abrundungen, wie beispielsweise Ellipsenabschnitte möglich. An der zum Kern 21 hin ausgerichteten Seite haben die Haken 25 Hinterschnitte, die zum Verhaken mit anderen Haken 25 eingreifender Partikel geeignet sind.

Um ein erfindungsgemäßes Knochenersatzmaterial zu bilden, liegen die Partikel bevorzugt aneinander anliegend aber nicht miteinander verhakt vor, so dass also die Haken 25 der Stifte 22 noch nicht ineinander greifen. Zudem können die Partikel mit einer Flüssigkeit gemischt als Schlämmung vorliegen. In der Flüssigkeit ist bevorzugt zumindest eine pharmazeutisch wirksame Substanz enthalten, die zur Bekämpfung der Infektion oder zum Anregen des Knochenwachstums geeignet ist. Alternativ oder zusätzlich können die Partikel mit einer solchen pharmazeutisch wirksamen Substanz beschichtet sein.

Das Knochenersatzmaterial kann geformt werden, indem die Partikel ineinander gedrückt werden. Dadurch verhaken oder rasten die Partikel miteinander und das Knochenersatzmaterial wird so in der gewünschten Form verfestigt. Die Partikel verbinden sich dabei untereinander in der Art, dass freie Zwischenräume zwischen den miteinander verbundenen Partikeln verbleiben, so dass der aus den Partikeln geformte Volumenköper offenporig ist. Die Partikel haben einen Durchmesser von etwa 8 mm, so dass die verbleibenden Poren einen freien Querschnitt im Bereich von etwa 0,8 mm aufweisen. Dieser Querschnitt reicht noch aus, damit sich in den Poren Knochenmaterial ausbilden beziehungsweise einwachsen kann. Der Körper mit seinen offenen Poren kann also als osteokonduktiv bezeichnet werden. Zur Förderung der Osteokonduktivität kann die Oberfläche der Partikel mit einer das Knochenwachstum fördernden Substanz beschichtet sein. Der aus den Partikeln geformte Volumenkörper ist daher als Knochenersatzmaterial gut geeignet.

Die Partikel sollten fest ineinander gedrückt werden, so dass der Volumenkörper formstabil ist. Die Partikel rasten dabei ineinander dadurch, dass die Haken 25 zwischen die Haken 25 verbundener Partikel gleiten oder die Stifte 22 verbundener Partikel elastisch verformen und durch die elastische Rückstellkraft der Stifte 22 und der Haken 25 die Haken 25 benachbarter Partikel zum Kern 21 hin gezogen werden. Es ist auch möglich, dass Kanten, Ecken oder Spitzen (nicht gezeigt) der Haken 25 die Stifte 22 oder die Haken 25 benachbarter Partikel in geringem Umfang plastisch verformen und dadurch eine Rastung der Partikel miteinander erfolgt.

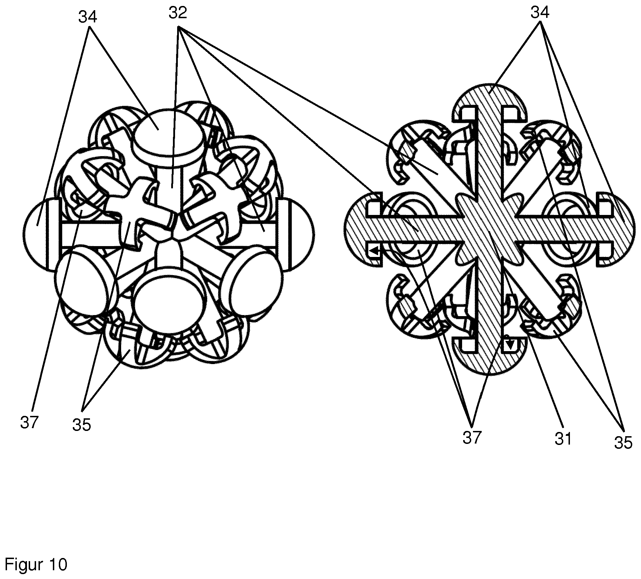

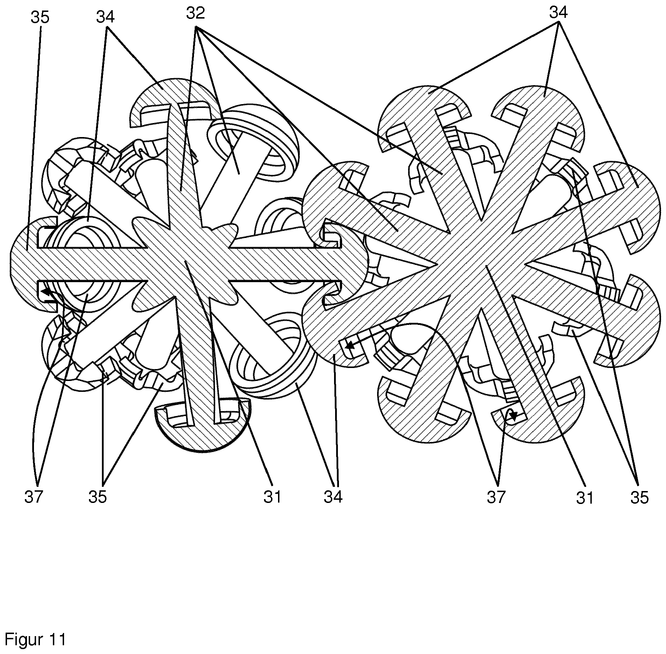

Figur 10 zeigt eine schematische Querschnittansicht (rechts) und schematische perspektivische Ansicht (links) zweier Partikel eines vierten alternativen erfindungsgemäßen Knochenersatzmaterials. Figur 11 zeigt eine schematische Querschnittansicht zweier miteinander verbundener Partikel des vierten erfindungsgemäßen Knochenersatzmaterials nach Figur 10. Die Partikel bestehen aus Tantal oder einer Tantallegierung, können aber auch aus anderen biokompatiblen Metallen oder biokompatiblen Metalllegierungen oder aus einem biokompatiblen Kunststoff gefertigt sein. Die Partikel werden mit einem CAM-Verfahren beziehungsweise einem 3D-Druckverfahren hergestellt. Alle geeigneten Rapid-Prototyping-Verfahren können auch zur Herstellung der Partikel verwendet werden.

Die Partikel sind aufgebaut aus einem Kern 31, der in dem geometrischen Zentrum des Partikels angeordnet ist, sowie zweiundzwanzig Stiften 32, die sich in verschiedene Richtungen radial vom Kern 31 weg erstrecken. An den Enden der Stifte 32 sind entweder Pilze 34 oder eine Gruppe von jeweils vier Haken 35 als Verbindungselemente an den ansonsten zylindrischen Stiften 32 angeordnet. Die Pilze 34 und die Haken 35 sind nach außen (vom Kern 31 weg) kugelförmig abgerundet geformt. Es sind auch andere Abrundungen, wie beispielsweise Ellipsenabschnitte möglich. An der zum Kern 31 hin ausgerichteten Seite haben die Pilze 34 Hinterschnitte 37. Ebenso weisen die Haken 35 Hinterschnitte auf. Die Hinterschnitte 37 der Pilze 34 und die Hinterschnitte der Haken 35 sind zum Verhaken mit anderen Pilzen 34 und Haken 35 eingreifender Partikel geeignet.

Um ein erfindungsgemäßes Knochenersatzmaterial zu bilden, liegen die Partikel bevorzugt aneinander anliegend aber nicht miteinander verhakt vor, so dass also die Pilze 34 und Haken 35 der Stifte 32 noch nicht ineinander greifen. Zudem können die Partikel mit einer Flüssigkeit gemischt als Schlämmung vorliegen. In der Flüssigkeit ist bevorzugt zumindest eine pharmazeutisch wirksame Substanz enthalten, die zur Bekämpfung der Infektion oder zum Anregen des Knochenwachstums geeignet ist. Alternativ oder zusätzlich können die Partikel mit einer solchen pharmazeutisch wirksamen Substanz beschichtet sein.

Das Knochenersatzmaterial kann geformt werden, indem die Partikel ineinander gedrückt werden. Dadurch verhaken oder rasten die Partikel miteinander, so wie das in der schematischen Querschnittansicht nach Figur 11 gezeigt ist. Die Haken 35 greifen dabei beispielsweise in die Hinterschnitte 37 der Pilze 34 ein. Um in diese Position zu gelangen, müssen die Stifte 32 elastisch zur Seite gebogen werden, indem ein mechanischer Druck auf die zu verbindenden Partikel ausgeübt wird. Beim elastischen Rückstellen der Stifte 32 werden die Haken 35 in die Hinterschnitte 37 der benachbarten Pilze 34 und/oder in die Hinterschnitte der benachbarten Haken 35 gedrückt. Bei der Ausführung nach den Figuren 10 und 11 sind die Längen und Durchmesser der Stifte 32 sowie die Form der Haken 35 und Pilze 34 derart aufeinander abgestimmt, dass die äußere Wölbung der Haken 35 gerade eine leichte elastische Deformation der Stifte 32 bewirkt, wenn die Spitzen der Haken 35 am Boden der Hinterschnitte 37 der verbundenen Pilze 34 und Haken 35 anliegen. Hierdurch wird eine Rastung der Haken 35 mit den Pilzen 34 erreicht, da die Haken 35 beziehungsweise die Pilze 34 nicht ohne Kraftaufwand (also nicht ohne weitere elastische Verformung der Stifte 32) tiefer ineinander geschoben werden können. Dies ist bei einer mehrfachen Verhakung beziehungsweise Rastung der Partikel durch mehrere Pilze 34 und/oder Haken 35 eines Partikels und/oder mehrerer Partikel mit einem anderen Partikel nicht mehr möglich Das Knochenersatzmaterial wird dadurch in der gewünschten Form verfestigt. Diese Art der Verbindung der Partikel ist in ähnlicher Form auf die anderen Ausführungsbeispiele nach den Figuren 1 bis 9 übertragbar.

Die Partikel verbinden sich dabei untereinander in der Art, dass freie Zwischenräume zwischen den miteinander verbundenen Partikeln verbleiben, so dass der aus den Partikeln geformte und verfestigte Volumenköper offenporig ist. Die Partikel haben einen Durchmesser von etwa 6 mm, so dass die verbleibenden Poren einen freien Querschnitt im Bereich von etwa 0,6 mm aufweisen. Dieser Querschnitt reicht noch aus, damit sich in den Poren Knochenmaterial ausbilden beziehungsweise einwachsen kann. Der Körper mit seinen offenen Poren kann also als osteokonduktiv bezeichnet werden. Zur Förderung der Osteokonduktivität kann die Oberfläche der Partikel mit einer das Knochenwachstum fördernden Substanz beschichtet sein. Der aus den Partikeln geformte Volumenkörper ist daher als Knochenersatzmaterial gut geeignet.

Die Partikel sollten fest ineinander gedrückt werden, so dass der Volumenkörper formstabil ist. Die Partikel rasten dabei dadurch ineinander, dass die Pilze 34 und Haken 35 zwischen die Pilze 34 und Haken 35 verbundener Partikel gleiten und dabei die Stifte 32 verbundener Partikel elastisch verformen. Durch die elastische Rückstellkraft der Stifte 32 können die Pilze 34 und Haken 35 in die Hinterschnitte 37 anderer Pilze 34 und Haken 35 benachbarter Partikel gezogen werden. Es ist auch möglich, dass Kanten, Ecken oder Spitzen (nicht gezeigt) der Haken 35 oder die Kanten der Pilze 34 die Stifte 32 oder die Pilze 34 oder Haken 35 benachbarter Partikel in geringem Umfang plastisch verformen und dadurch eine Rastung der Partikel miteinander erfolgt.

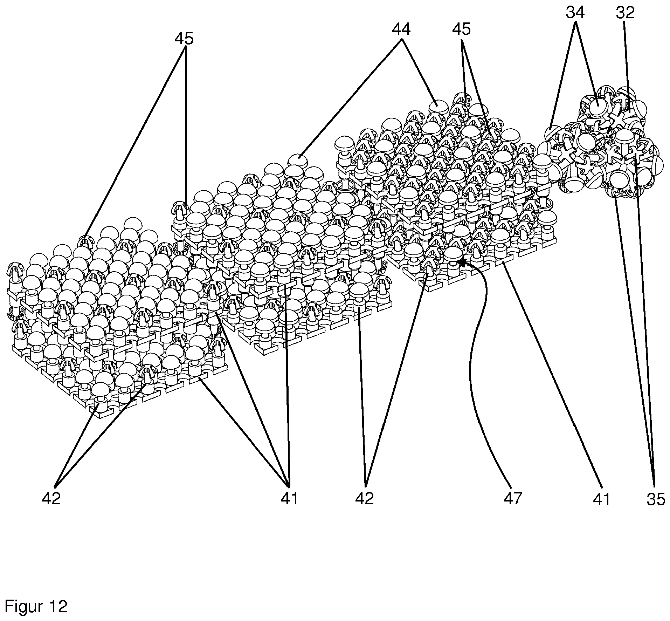

Figur 12 zeigt eine schematische perspektivische Ansicht dreier miteinander verbundener Partikel des vierten erfindungsgemäßen Knochenersatzmaterials nach Figur 10 und mehrere Platten eines Knochenersatzmaterials, die mit den Partikeln verbunden werden können. Die Platten weisen ein tragendes Flächengebilde 41 mit einer Vielzahl von Durchbrechungen auf, wobei zwischen den Durchbrechungen Stifte 42 auf dem Flächengebilde 41 angeordnet sind, die in Pilzen 44 oder in Gruppen von jeweils vier Haken 45 als Verbindungselemente 44, 45 enden. Die Stifte 42 mit den Pilzen 44 oder den Haken 45 sind analog den Stiften 32 mit den Pilzen 34 und Haken 35 der Partikel aufgebaut und weisen demzufolge umlaufende Nuten 47 und Hinterschnitte auf. Im Unterschied zu den Stiften 32 der Partikel erstrecken sich die Stifte 42 nicht radial von einem Kern 31 sondern senkrecht von dem Flächengebilde 41. Die Platten können dabei Stifte 42 auf beiden Seiten des Flächengebildes 41 aufweisen oder nur auf einer Seite des Flächengebildes 41.

Die Platten können alternativ auch andere Stifte und Verbindungselemente aufweisen, wie die zu den Partikeln der Figuren 1 bis 9 beschriebenen. Bevorzugt werden die Stifte und Verbindungselemente der Platten auf die Stifte und Verbindungselemente der Partikel abgestimmt, damit eine gleichmäßige Stabilität erreicht werden kann. Die Materialien, aus denen die Platten hergestellt werden, können die gleichen sein, wie die der Partikel, ebenso können die gleichen Herstellungsverfahren verwendet werden. Die Platten sind aufgrund der Größe (Dicke etwa 1 mm bis 10 mm) verformbar und an die zu behandelnde Knochenoberfläche anpassbar.

Die Platten können über Befestigungsmittel (nicht gezeigt) in Form von Spitzen oder Schrauben mit dem Knochen eines Patienten verbunden werden. Anschließend können weitere Platten oder auf den Platten die Partikel eines erfindungsgemäßen Knochenersatzmaterials befestigt werden. Die Partikel und die Platten verbinden sich dabei untereinander in der Art, dass freie Zwischenräume zwischen den miteinander verbundenen Partikeln und Platten verbleiben, so dass der aus den Partikeln und Platten geformte und verfestigte Volumenköper offenporig ist. Die freien Querschnitte der offenporigen Struktur muss noch ausreichen, damit sich in den Poren Knochenmaterial ausbilden beziehungsweise einwachsen kann. Der aus den Platten und Partikeln geformte Körper mit seinen offenen Poren kann also als osteokonduktiv bezeichnet werden. Zur Förderung der Osteokonduktivität kann die Oberfläche der Platten mit einer das Knochenwachstum fördernden Substanz beschichtet sein. Der aus den Partikeln und Platten geformte Volumenkörper ist daher als Knochenersatzmaterial gut geeignet.

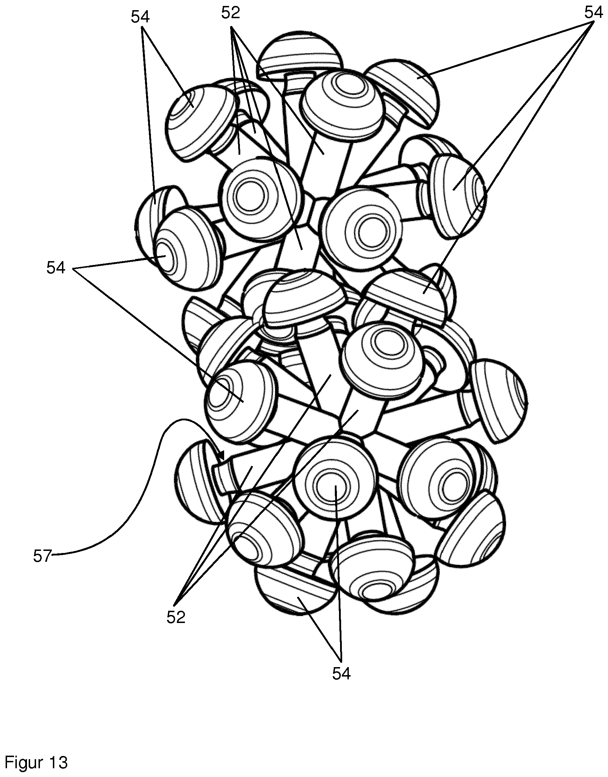

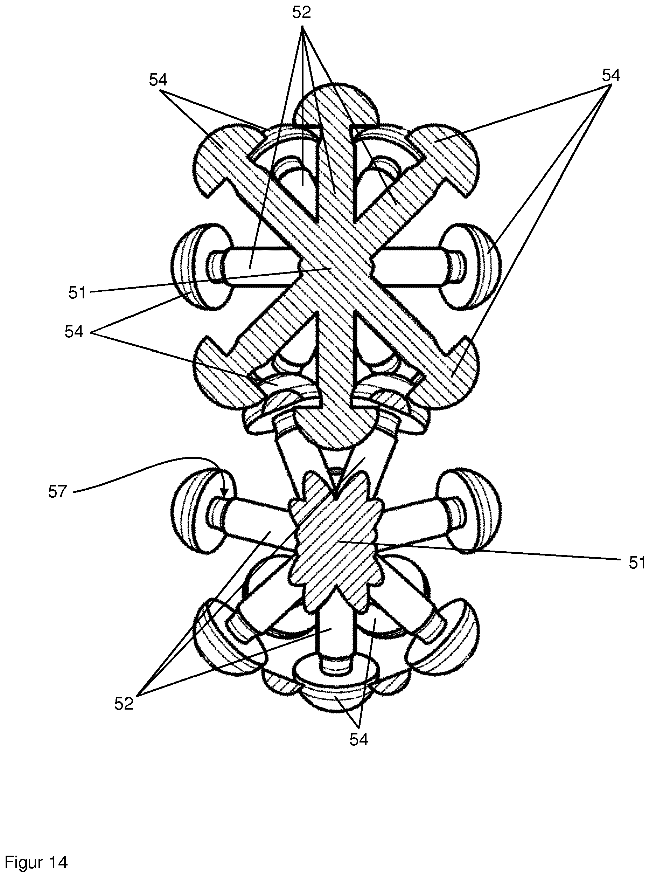

Figur 13 zeigt eine schematische perspektivische Ansicht zweier Partikel eines fünften alternativen erfindungsgemäßen Knochenersatzmaterials. Figur 14 zeigt eine schematische Querschnittansicht der verbundenen Partikel des fünften erfindungsgemäßen Knochenersatzmaterials nach Figur 13. Die Partikel bestehen aus Tantal oder einer Tantallegierung oder einem anderen biokompatiblen Metall oder einer anderen biokompatiblen Metalllegierung, können aber auch aus einem biokompatiblen Kunststoff gefertigt sein. Die Partikel werden mit einem CAM-Verfahren beziehungsweise einem 3D-Druckverfahren hergestellt. Alle geeigneten Rapid-Prototyping-Verfahren können auch zur Herstellung der Partikel verwendet werden.

Die Partikel sind aufgebaut aus einem Kern 51, der in dem geometrischen Zentrum des Partikels angeordnet ist, sowie zwanzig Stiften 52, die sich in verschiedene Richtungen radial vom Kern 51 weg erstrecken. Der Partikel hat ikosaedrische Symmetrie, so dass die Enden der Stifte 52 auf einer Kugeloberfläche um das Zentrum des Kerns 51 angeordnet sind. An den Enden der Stifte 52 sind Pilze 54 als Verbindungselemente an den Stiften 52 angeordnet. Unterhalb der Pilze 54 weisen die Stifte 52 eine Umlaufende Nut 57 als zusätzliches Verbindungsmittel 57 auf. Die Stifte 52 sind bis auf die Pilze 54 und die Nut 57 zylindrisch. Die Pilze 54 sind nach außen (vom Kern 51 weg) kugelförmig abgerundet geformt. Es sind auch andere Abrundungen, wie beispielsweise Ellipsenabschnitte möglich. Die Nut 57 der Pilze 54 sind zum Rasten mit anderen Pilzen 54 eingreifender Partikel geeignet. Die Pilze 54 bilden so Rastmittel 54 und die Nuten 57 bilden Gegenrastmittel 57, die miteinander rasten, wenn die Partikel ineinander gepresst werden.

Um ein erfindungsgemäßes Knochenersatzmaterial zu bilden, liegen die Partikel bevorzugt aneinander anliegend aber nicht miteinander verbunden vor, so dass also die Pilze 54 und Nuten 57 der Stifte 52 noch nicht ineinander greifen. Zudem können die Partikel mit einer Flüssigkeit gemischt als Schlämmung vorliegen. In der Flüssigkeit ist bevorzugt zumindest eine pharmazeutisch wirksame Substanz enthalten, die zur Bekämpfung der Infektion oder zum Anregen des Knochenwachstums geeignet ist. Alternativ oder zusätzlich können die Partikel mit einer solchen pharmazeutisch wirksamen Substanz beschichtet sein.

Das Knochenersatzmaterial kann geformt werden, indem die Partikel ineinander gedrückt werden. Dadurch rasten die Partikel miteinander, so wie das in den Figuren 13 und 14 gezeigt ist und in der schematischen Querschnittansicht nach Figur 14 gut zu erkennen ist. Die Ränder der Pilze 54 greifen dabei in die Nuten 57 der Stifte 52 ein. Um in diese Position zu gelangen, müssen die Stifte 52 elastisch zur Seite gebogen werden, indem ein mechanischer Druck auf die zu verbindenden Partikel ausgeübt wird. Beim elastischen Rückstellen der Stifte 52 werden die Pilze 54 in die Nuten 57 der benachbarten Pilze 54 gedrückt. Bei der Ausführung nach den Figuren 13 und 14 sind die Längen und Durchmesser der Stifte 52 sowie die Form der Nuten 57 und Pilze 54 derart aufeinander abgestimmt, dass die äußere Wölbung der Pilze 54 Genau in die Nuten 57 der Stifte 52 passt. Hierdurch wird eine Rastung der Pilze 54 mit den Nuten 57 erreicht, da die Pilze 54 nicht ohne Kraftaufwand (also nicht ohne weitere elastische Verformung der Stifte 52) tiefer ineinander geschoben werden können. Dies ist bei einer mehrfachen Rastung der Partikel durch mehrere Pilze 54 und Nuten 57 eines Partikels und/oder mehrerer Partikel mit einem anderen Partikel nicht mehr ohne weiteres möglich Das Knochenersatzmaterial wird dadurch in der gewünschten Form verfestigt. Diese Art der Verbindung der Partikel ist in ähnlicher Form auf die anderen Ausführungsbeispiele nach den Figuren 1 bis 11 übertragbar.

Die Partikel verbinden sich dabei untereinander in der Art, dass freie Zwischenräume zwischen den miteinander verbundenen Partikeln verbleiben, so dass der aus den Partikeln geformte und verfestigte Volumenköper offenporig ist. Die Partikel haben einen Durchmesser von etwa 5 mm, so dass die verbleibenden Poren einen freien Querschnitt im Bereich von etwa 0,5 mm aufweisen. Dieser Querschnitt reicht noch aus, damit sich in den Poren Knochenmaterial ausbilden beziehungsweise einwachsen kann. Der Körper mit seinen offenen Poren kann also als osteokonduktiv bezeichnet werden. Zur Förderung der Osteokonduktivität kann die Oberfläche der Partikel mit einer das Knochenwachstum fördernden Substanz beschichtet sein. Der aus den Partikeln geformte Volumenkörper ist daher als Knochenersatzmaterial gut geeignet.

Die Partikel sollten fest ineinander gedrückt werden, so dass der Volumenkörper formstabil ist. Die Partikel rasten dabei dadurch ineinander, dass die Pilze 54 zwischen die Pilze 54 verbundener Partikel gleiten und dabei die Stifte 52 verbundener Partikel elastisch verformen. Durch die elastische Rückstellkraft der Stifte 52 können die Pilze 54 in die Nuten 57 anderer Stifte 52 benachbarter Partikel gezogen werden. Es ist auch möglich, dass Kanten der Pilze 54 die Stifte 52 benachbarter Partikel in geringem Umfang plastisch verformen und dadurch eine Rastung der Partikel miteinander erfolgt.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 11, 21, 31, 51: Kern
- 2, 12, 22, 32, 52: Stift
- 4, 14, 34: Pilz / Verbindungselement
- 6, 16: Greiffläche
- 25, 35: Haken / Verbindungselement
- 37: Hinterschnitt
- 41: Flächengebilde
- 42: Stift
- 44: Pilz / Verbindungselement
- 45: Haken / Verbindungselement
- 47: Nut
- 54: Pilz / Verbindungmittel / Rastmittel
- 57: Nut / Gegenrastmittel

## Patentansprüche

1. Partikuläres alloplastisches Knochenersatzmaterial aufweisend eine Vielzahl von Partikeln, wobei die Partikel einen Kern (1, 11, 21, 31, 51) und mindestens sechs sich vom Kern (1, 11, 21, 31, 51) erstreckende Stifte (2, 12, 22, 32, 52) aufweisen, wobei die Stifte (2, 12, 22, 32, 52) mindestens ein Verbindungselement (4, 14, 25, 34, 35, 37, 54, 57) aufweisen und wobei die Stifte (2, 12, 22, 32, 52) elastisch verformbar sind, so dass durch Zusammenpressen mehrerer Partikel die Verbindungselemente (4, 14, 25, 34, 35, 37, 54, 57) verschiedener Partikel miteinander verhaken und/oder rasten und die miteinander verhakten und/oder gerasteten Partikel einen offenporigen Körper aus miteinander verhakten und/oder gerasteten Partikeln bilden, **dadurch gekennzeichnet dass**
jeder der Stifte (2, 12, 22, 32, 52) mindestens ein Verbindungselement (4, 14, 25, 34, 35, 37, 54, 57) aufweist.

2. Knochenersatzmaterial nach Anspruch 1, **dadurch gekennzeichnet, dass**
die Verbindungselemente (4, 14, 25, 34, 35, 37, 54, 57) Pilze (4, 14, 34, 54), Haken (25, 35), Hinterschnitte (37) und/oder Rastelemente (54, 57) sind, vorzugsweise Pilze (4, 14, 34, 54), Haken (25, 35), Hinterschnitte (37), Rastmittel (54) und/oder Gegenrastmittel (57) sind.

3. Knochenersatzmaterial nach einem der vorangehenden Ansprüche, **dadurch gekennzeichnet, dass**
die Verbindungselemente (4, 14, 25, 34, 35, 37, 54, 57) an der Mantelfläche der Stifte (2, 12, 22, 32, 52) ausgebildet sind.

4. Knochenersatzmaterial nach einem der vorangehenden Ansprüche, **dadurch gekennzeichnet, dass**
zumindest eines der zumindest einen Verbindungselemente (4, 14, 25, 34, 35, 37, 54, 57) pro Stift (2, 12, 22, 32, 52) stumpf-kegelförmig ausgebildet ist, wobei die Längsachsen der Stifte (2, 12, 22, 32, 52) die Längsachsen der Kegel bilden und wobei der Mantel der Kegel nach der vom Kern (1, 11, 21, 31, 51) abgewandten Außenseite gerichtet ist.

5. Knochenersatzmaterial nach einem der vorangehenden Ansprüche, **dadurch gekennzeichnet, dass**
zumindest eines der zumindest einen Verbindungselemente (4, 14, 25, 34, 35, 37, 54, 57) pro Stift (2, 12, 22, 32, 52) als Haken (25, 35) oder als Pilzkopf (4, 14, 34, 54) ausgebildet ist.

6. Knochenersatzmaterial nach einem der vorangehenden Ansprüche, **dadurch gekennzeichnet, dass**
die Stifte (2, 12, 22, 32, 52) zwischen dem Kern (1, 11, 21, 31, 51) und zumindest einem der zumindest einen Verbindungselemente (4, 14, 25, 34, 35, 37, 54) eine umlaufende Nut (57) als zusätzliches Verbindungselement (57) enthalten, in welche Verbindungselemente (4, 14, 25, 34, 35, 37, 54) anderer Partikel einhaken oder einrasten können, bevorzugt derart einrasten, dass keine weitere Bewegung der Verbindungselemente (4, 14, 25, 34, 35, 37, 54, 57) entlang der Stifte (2, 12, 22, 32, 52) möglich ist.

7. Knochenersatzmaterial nach einem der vorangehenden Ansprüche, **dadurch gekennzeichnet, dass**
zumindest zwei Verbindungselemente (4, 14, 25, 34, 35, 37, 54, 57) hintereinander auf der Mantelfläche der Stifte (2, 12, 22, 32, 52) angeordnet sind, besonders bevorzugt zumindest drei Verbindungselemente (4, 14, 25, 34, 35, 37, 54, 57) hintereinander auf der Mantelfläche der Stifte (2, 12, 22, 32, 52) angeordnet sind.

8. Knochenersatzmaterial nach einem der vorangehenden Ansprüche, **dadurch gekennzeichnet, dass**
die Partikel aus biokompatiblem Kunststoff, Edelstahl, Titan, einer Titanlegierung, Tantal, einer Tantallegierung oder aus Kompositen dieser Materialien gebildet sind.

9. Knochenersatzmaterial nach einem der vorangehenden Ansprüche, **dadurch gekennzeichnet, dass**
benachbarte Stifte (2, 12, 22, 32, 52) eines Partikels einen derartigen Abstand zueinander aufweisen, dass nach einer elastischer Deformation aufgrund einer Verhakung und/oder Rastung mit einem Verbindungselement (4, 14, 25, 34, 35, 37, 54, 57) eines anderen Partikels die Stifte (2, 12, 22, 32, 52) des Partikels mindestens zwei Verhakungen und/oder Rastungen mit zwei weiteren Partikeln ermöglichen, bevorzugt mindestens drei Verhakungen und/oder Rastungen mit drei weiteren Partikeln ermöglichen, ganz besonders bevorzugt mehr als drei Verhakungen und/oder Rastungen mit mehr als drei weiteren Partikeln ermöglichen.

10. Knochenersatzmaterial nach einem der vorangehenden Ansprüche, **dadurch gekennzeichnet, dass**
die Partikel in einer wässrigen oder nichtwässrigen Lösung von biokompatiblen Polymeren und/oder Oligomeren suspendiert sind und mit der Lösung eine pastöse Masse bilden oder die Partikel in einer bei Raumtemperatur hydrophoben, niedermolekularen Flüssigkeit suspendiert sind und mit der Flüssigkeit eine pastöse Masse bilden.

11. Knochenersatzmaterial nach einem der vorangehenden Ansprüche, **dadurch gekennzeichnet, dass**
die Partikel mindestens vierzehn sich vom Kern (1, 11, 21, 31, 51) erstreckende Stifte (2, 12, 22, 32, 52) aufweisen, bevorzugt mindestens zwanzig sich vom Kern (1, 11, 21, 31, 51) erstreckende Stifte (2, 12, 22, 32, 52) aufweisen, besonders bevorzugt zwischen zwanzig und fünfzig sich vom Kern (1, 11, 21, 31, 51) erstreckende Stifte (2, 12, 22, 32, 52) aufweisen, ganz besonders bevorzugt zwischen dreißig und vierzig sich vom Kern (1, 11, 21, 31, 51) erstreckende Stifte (2, 12, 22, 32, 52) aufweisen.

12. Knochenersatzmaterial nach einem der vorangehenden Ansprüche, **dadurch gekennzeichnet, dass**
die Poren des aus den Partikeln gebildeten offenporigen Körpers interkonnektierend und osteokonduktiv sind, wobei vorzugsweise die Poren einen freien Querschnitt zwischen 0,1 mm und 1 mm aufweisen, besonders bevorzugt zwischen 0,25 mm und 0,9 mm.

13. Knochenersatzmaterial nach einem der vorangehenden Ansprüche, **dadurch gekennzeichnet, dass**
das Knochenersatzmaterial neben den Partikeln zusätzlich zumindest eine Platte aufweist, wobei die zumindest eine Platte ein Flächengebilde (41) aufweist und eine Mehrzahl von sich aus dem Flächengebilde (41) der zumindest einen Platte heraus erstreckende Stifte (42) aufweist, wobei die Stifte (42) jeweils mindestens ein Verbindungselement (44, 45) aufweisen, das analog der Verbindungselemente (4, 14, 25, 34, 35, 37, 54, 57) der Partikel ausgebildet ist, so dass die Partikel mit der zumindest einen Platte durch Aufeinanderpressen die Verbindungselemente verschiedener Platten und Partikel miteinander verhaken und/oder rasten und die miteinander verhakten und/oder gerasteten Platten und Partikel einen offenporigen Körper aus miteinander verhakten und/oder gerasteten Platten und Partikeln bilden.

14. Verfahren zum Formen eines Körpers aus einem partikulären alloplastischen Knochenersatzmaterial nach einem der vorangehenden Ansprüche, bei dem die Partikel gegeneinander gedrückt werden, wobei die Partikel dadurch miteinander verhaken und/oder rasten und einen offenporigen Körper ausbilden.

15. Verfahren nach Anspruch 14, **dadurch gekennzeichnet, dass**
die Partikel mit einem porösen Volumenkörper eines zweiten Knochenersatzmaterials verbunden werden, indem die Verbindungselemente (4, 14, 25, 34, 35, 37, 54, 57) mit den Poren des zweiten Knochenersatzmaterials rasten und/oder verhaken, und/oder die Partikel mit einem flächigen dritten Knochenersatzmaterial verbunden werden, das eine Vielzahl von Stiften (42) mit Verbindungelementen (44, 45) aufweist, wobei bevorzugt die Stifte (42) und die Verbindungselemente (44, 45) des flächigen dritten Knochenersatzmaterials die Merkmale der Stifte (2, 12, 22, 32, 52) und Verbindungselemente (4, 14, 25, 34, 35, 37, 54, 57) der Partikel des Knochenersatzmaterials nach einem der Ansprüche 1 bis 13 aufweisen.

## Claims

1. Particulate alloplastic bone replacement material comprising a multitude of particles, whereby the particles comprise a core (1, 11, 21, 31, 51) and at least six pins (2, 12, 22, 32, 52) extending from the core (1, 11, 21, 31,51), whereby the pins (2, 12, 22, 32, 52) comprise at least one connecting element (4, 14, 25, 34, 35, 37, 54, 57), and whereby the pins (2, 12, 22, 32, 52) are deformable elastically such that, upon multiple particles being pressed together, the connecting elements (4, 14, 25, 34, 35, 37, 54, 57) of different particles interlock with and/or snap into each other and the particles that are interlocked with and/or snapped into each other form an open-pored body of particles that are interlocked with and/or snapped into each other, **characterised in that** each of the pins (2, 12, 22, 32, 52) comprises at least one connecting element (4, 14, 25, 34, 35, 37, 54, 57).

2. Bone replacement material according to claim 1, **characterised in that**
the connecting elements (4, 14, 25, 34, 35, 37, 54, 57) are mushrooms (4, 14, 34, 54), hooks (25, 35), undercuts (37) and/or snap-in elements (54, 57), preferably mushrooms (4, 14, 34, 54), hooks (25, 35), undercuts (37), snap-in means (54) and/or opposite snap-in means (57).

3. Bone replacement material according to any one of the preceding claims,
**characterised in that**
the connecting elements (4, 14, 25, 34, 35, 37, 54, 57) are provided on the jacket surface of the pins (2, 12, 22, 32, 52).

4. Bone replacement material according to any one of the preceding claims,
**characterised in that**
at least one of the at least one connecting elements (4, 14, 25, 34, 35, 37, 54, 57) per pin (2, 12, 22, 32, 52) has a truncated cone shape, whereby the longitudinal axes of the pins (2, 12, 22, 32, 52) form the longitudinal axes of the cones and whereby the jacket of the cones faces toward the outer side that faces away from the core (1, 11, 21, 31, 51).

5. Bone replacement material according to any one of the preceding claims,
**characterised in that**
at least one of the at least one connecting elements (4, 14, 25, 34, 35, 37, 54, 57) per pin (2, 12, 22, 32, 52) is provided in the form of a hook (25, 35) or as a mushroom head (4, 14, 34, 54).

6. Bone replacement material according to any one of the preceding claims,
**characterised in that**
the pins (2, 12, 22, 32, 52) contain a circumferential groove (57) as additional connecting element (57) between the core (1, 11, 21, 31, 51) and at least one of the at least one connecting elements (4, 14, 25, 34, 35, 37, 54), whereby connecting elements (4, 14, 25, 34, 35, 37, 54) of other particles can interlock with or snap into said groove (57), preferably snap-in appropriately such that no further motion of the connecting elements (4, 14, 25, 34, 35, 37, 54) along the pins (2, 12, 22, 32, 52) is possible.

7. Bone replacement material according to any one of the preceding claims,
**characterised in that**
at least two connecting elements (4, 14, 25, 34, 35, 37, 54, 57) are arranged in succession on the jacket surface of the pins (2, 12, 22, 32, 52), particularly preferably at least three connecting elements (4, 14, 25, 34, 35, 37, 54, 57) are arranged in succession on the jacket surface of the pins (2, 12, 22, 32, 52).

8. Bone replacement material according to any one of the preceding claims,
**characterised in that**
the particles are made from biocompatible plastic material, stainless steel, titanium, a titanium alloy, tantalum, a tantalum alloy or composites of said materials.

9. Bone replacement material according to any one of the preceding claims,
**characterised in that**
neighbouring pins (2, 12, 22, 32, 52) of a particle are situated at an appropriate distance from each other such that the pins (2, 12, 22, 32, 52) of the particle, after elastic deformation due to interlocking and/or snapping into a connecting element (4, 14, 25, 34, 35, 37, 54, 57) of another particle, enable at least two interlocks and/or snap-in connections to two other particles, preferably enable at least three interlocks and/or snap-in connections to three other particles, particularly preferably enable more than three interlocks and/or snap-in connections to more than three other particles.

10. Bone replacement material according to any one of the preceding claims,
**characterised in that**
the particles are suspended in an aqueous or non-aqueous solution of biocompatible polymers and/or oligomers, and the particles and the solution, together, form a pasty mass or
the particles are suspended in a low-molecular liquid that is hydrophobic at room temperature, and the particles and the liquid, together, form a pasty mass.

11. Bone replacement material according to any one of the preceding claims,
**characterised in that**
the particles comprise at least fourteen pins (2, 12, 22, 32, 52) extending from the core (1, 11, 21, 31, 51), preferably at least twenty pins (2, 12, 22, 32, 52) extending from the core (1, 11, 21, 31, 51), particularly preferably between twenty and fifty pins (2, 12, 22, 32, 52) extending from the core (1, 11, 21, 31, 51), even more particularly preferably between thirty and forty pins (2, 12, 22, 32, 52) extending from the core (1, 11, 21, 31, 51).

12. Bone replacement material according to any one of the preceding claims,
**characterised in that**
The pores of the open-pored body formed from the particles are interconnecting and osteoconductive, whereby the pores preferably have a free cross-section between 0.1 mm and 1 mm, particularly preferably between 0.25 mm and 0.9 mm.

13. Bone replacement material according to any one of the preceding claims,
**characterised in that**
the bone replacement material comprises at least one plate aside from the particles, whereby the at least one plate comprises a planar structure (41) and a plurality of pins (42) extending out of the planar structure (41) of the at least one plate, whereby the pins (42) each comprise at least one connecting element (44, 45) that is designed in analogous manner to the connecting elements (4, 14, 25, 34, 35, 37, 54, 57) of the particles such that the particles and the at least one plate interlock with and/or snap into each other by pressing the connecting elements of various plates and particles onto each other, and whereby the plates and particles that are interlocked with and/or snapped into each other form an open-pored body of plates and particles that are interlocked with and/or snapped into each other.

14. Method for forming a body made of a particulate alloplastic bone replacement material according to any one of the preceding claims, in which the particles are pushed against each other, whereby the particles interlock with and/or snap into each other and form an open-pored body.

15. Method according to claim 14, **characterised in that**
the particles are being connected to a porous three-dimensional body of a second bone replacement material by snapping-in and/or interlocking the connecting elements (4, 14, 25, 34, 35, 37, 54, 57) with the pores of the second bone replacement material, and/or the particles are being connected to a planar third bone replacement material comprising a plurality of pins (42) comprising connecting elements (44, 45), whereby, preferably, the pins (42) and the connecting elements (44, 45) of the planar third bone replacement material comprise the features of the pins (2, 12, 22, 32, 52) and connecting elements (4, 14, 25, 34, 35, 37, 54, 57) of the particles of the bone replacement material according to anyone of the claims 1 to 13.

## Revendications

1. Matériau de substitution d'os alloplastique particulaire présentant une pluralité de particules, où les particules présentent un noyau (1, 11, 21, 31, 51) et au moins six tiges (2, 12, 22, 32, 52) s'étendant à partir du noyau (1, 11, 21, 31, 51), où les tiges (2, 12, 22, 32, 52) présentent au moins un élément de liaison (4, 14, 25, 34, 35, 37, 54, 57) et où les tiges (2, 12, 22, 32, 52) peuvent se déformer de manière élastique de sorte que les éléments de liaison (4, 14, 25, 34, 35, 37, 54, 57) de diverses particules s'accrochent et/ou s'encliquètent les unes dans les autres du fait d'une compression de plusieurs particules, et les particules accrochées et/ou encliquetées les unes dans les autres forment un corps à pores ouverts constitué de particules accrochées et encliquetées ensemble, **caractérisé en ce que** chacune des tiges (2, 12, 22, 32, 52) présente au moins un élément de liaison (4, 14, 25, 34, 35, 37, 54, 57).

2. Matériau de substitution d'os selon la revendication 1, **caractérisé en ce que**
les éléments de liaison (4, 14, 25, 34, 35, 37, 54, 57) sont des pointes en champignons (4, 14, 34, 54), des crochets (25, 35), des contre-dépouilles (37) et/ou des éléments de butée (54, 57), sont de préférence des pointes en champignons (4, 14, 34, 54), des crochets (25, 35), des contre-dépouilles (37) et/ou des éléments de butée (54) et des contrebutées (57).

3. Matériau de substitution d'os selon l'une des revendications précédentes, **caractérisé en ce que**
les éléments de liaison (4, 14, 25, 34, 35, 37, 54, 57) sont formés sur la surface d'enveloppe des tiges (2, 12, 22, 32, 52).

4. Matériau de substitution d'os selon l'une des revendications précédentes, **caractérisé en ce**
**qu'**au moins un des au moins un élément de liaison (4, 14, 25, 34, 35, 37, 54, 57) par tige (2, 12, 22, 32, 52) est conçu en forme de cône tronqué, où les axes longitudinaux des tiges (2, 12, 22, 32, 52) forment les axes longitudinaux des cônes et où l'enveloppe des cônes est orientée vers la face extérieure opposée au noyau (1, 11, 21, 31, 51).

5. Matériau de substitution d'os selon l'une des revendications précédentes, **caractérisé en ce**
**qu'**au moins un des au moins un élément de liaison (4, 14, 25, 34, 35, 37, 54, 57) par tige (2, 12, 22, 32, 52) est conçu en forme de crochet (25, 35) ou en tant que pointe en champignon (4, 14, 34, 54).

6. Matériau de substitution d'os selon l'une des revendications précédentes, **caractérisé en ce que**
les tiges (2, 12, 22, 32, 52) contiennent une rainure circonférentielle (57), en tant qu'élément de liaison (57) entre le noyau (1, 11, 21, 31, 51) et au moins un des au moins un élément de liaison (4, 14, 25, 34, 35, 37, 54), dans laquelle des éléments de liaison (4, 14, 25, 34, 35, 37, 54) d'autres particules peuvent s'accrocher ou s'encliqueter, de préférence, s'encliqueter de sorte qu'un nouveau déplacement des éléments de liaison (4, 14, 25, 34, 35, 37, 54, 57) le long des tiges (2, 12, 22, 32, 52) n'est pas possible.

7. Matériau de substitution d'os selon l'une des revendications précédentes, **caractérisé en ce**
**qu'**au moins deux éléments de liaison (4, 14, 25, 34, 35, 37, 54, 57) sont disposés l'un derrière l'autre sur la surface d'enveloppe des tiges (2, 12, 22, 32, 52), de manière particulièrement préférée, qu'au moins trois éléments de liaison (4, 14, 25, 34, 35, 37, 54, 57) sont disposés les uns derrière les autres sur la surface d'enveloppe des tiges (2, 12, 22, 32, 52).

8. Matériau de substitution d'os selon l'une des revendications précédentes, **caractérisé en ce que**
les particules sont formées à base d'une matière plastique biocompatible, d'acier inoxydable, de titane, d'un alliage de titane, de tantale, d'un alliage de tantale ou à base de composites de ces matériaux.

9. Matériau de substitution d'os selon l'une des revendications précédentes, **caractérisé en ce que**
des tiges (2, 12, 22, 32, 52) voisines d'une particule présentent un espace l'une par rapport à l'autre tel qu'après une déformation élastique en raison d'un accrochage et/ou d'un encliquetage avec un élément de liaison (4, 14, 25, 34, 35, 37, 54, 57) d'une autre particule, les tiges (2, 12, 22, 32, 52) de la particule permettent au moins deux accrochages et/ou encliquetages avec deux autres particules, de préférence, au moins trois accrochages et/ou encliquetages avec trois autres particules, de manière particulièrement préférée, plus de trois accrochages et/ou encliquetages avec plus de trois autres particules.

10. Matériau de substitution d'os selon l'une des revendications précédentes, **caractérisé en ce que**
les particules sont en suspension dans une solution aqueuse ou non aqueuse de polymères et/ou d'oligomères biocompatibles et forment une masse pâteuse avec la solution, ou les particules sont en suspension dans un liquide à bas poids moléculaire hydrophobe à la température ambiante et forment une masse pâteuse avec le liquide.

11. Matériau de substitution d'os selon l'une des revendications précédentes, **caractérisé en ce que**
les particules présentent au moins quatorze tiges (2, 12, 22, 32, 52) s'étendant à partir du noyau (1, 11, 21, 31, 51), présentent de préférence au moins vingt tiges (2, 12, 22, 32, 52) s'étendant à partir du noyau (1, 11, 21, 31, 51), de manière particulièrement préférée, présentent entre vingt et cinquante tiges (2, 12, 22, 32, 52) s'étendant à partir du noyau (1, 11, 21, 31, 51), de manière particulièrement préférée présentent entre trente et quarante tiges (2, 12, 22, 32, 52) s'étendant à partir du noyau (1, 11, 21, 31, 51).

12. Matériau de substitution d'os selon l'une des revendications précédentes, **caractérisé en ce que**
les pores du corps à pores ouverts formé à partir des particules sont interconnectés et ostéo-conducteurs, où, de préférence, les pores présentent une section transversale ouverte entre 0,1 mm et 1 mm, de manière particulièrement préférée entre 0,25 mm et 0,9 mm.

13. Matériau de substitution d'os selon l'une des revendications précédentes, **caractérisé en ce que**
le matériau de substitution d'os présente en complément des particules au moins une plaque, où l'au moins une plaque présente une structure de surface (41) et une pluralité de tiges (42) s'étendant hors de la structure de surface (41) de l'au moins une plaque, où les tiges (42) présentent respectivement au moins un élément de liaison (44, 45) qui est conçu de manière analogue aux éléments de liaison (4, 14, 25, 34, 35, 37, 54, 57) des particules de sorte que les particules s'accrochent et/ou s'encliquètent ensemble avec l'au moins une plaque par la compression des éléments de liaison de diverses plaques les unes au dessus des autres et des particules, et les plaques accrochées et/ou encliquetées ensemble et les particules forment un corps à pores ouverts constitué de plaques accrochées et/ou encliquetées ensemble et de particules.

14. Procédé de formation d'un corps à base d'un matériau de substitution d'os alloplastique particulaire selon l'une des revendications précédentes, chez lequel les particules sont pressées les unes contre les autres, ce par quoi les particules s'accrochent et/ou s'encliquètent entre elles et forment un corps à pores ouverts.

15. Procédé selon la revendication 14, **caractérisé en ce que**
les particules sont reliées avec un corps de volume poreux d'un deuxième matériau de substitution d'os, **en ce que** les éléments de liaison (4, 14, 25, 34, 35, 37, 54, 57) s'encliquètent et/ou s'accrochent avec les pores du deuxième matériau de substitution d'os, et/ou les particules sont reliées avec un troisième matériau de substitution d'os plat qui présente une pluralité de tiges (42) avec des éléments de liaison (44, 45), de préférence, les tiges (42) et les éléments de liaison (44, 45) du troisième matériau de substitution d'os plat présentent les caractéristiques des tiges (2, 12, 22, 32, 52) et des éléments de liaison (4, 14, 25, 34, 35, 37, 514, 57) des particules du matériau de substitution d'os selon l'une des revendications 1 à 13.
